(19) Europäisches Patentamt
European Patent Office
Office européen des brevets

(11) EP 2 723 306 B1

(12) **EUROPEAN PATENT SPECIFICATION**

(45) Date of publication and mention of the grant of the patent:
**28.12.2016 Bulletin 2016/52**

(21) Application number: **12730899.7**

(22) Date of filing: **22.06.2012**

(51) Int Cl.:
*A61K 8/19* (2006.01) *A61K 8/27* (2006.01)
*A61K 8/365* (2006.01) *A61K 8/368* (2006.01)
*A61K 8/49* (2006.01) *A61Q 5/10* (2006.01)
*A61K 8/34* (2006.01) *A61K 8/29* (2006.01)

(86) International application number:
**PCT/EP2012/062102**

(87) International publication number:
**WO 2012/175683 (27.12.2012 Gazette 2012/52)**

(54) **HAIR DYEING PROCESS USING AT LEAST ONE ORTHO-DIPHENOL, A MANGANESE OR ZINC SALT, HYDROGEN PEROXIDE, (BI)CARBONATE, AN ALKALINE AGENT AND A TITANIUM OR SCANDIUM SALT**

HAARFÄRBEVERFAHREN MIT MINDESTENS EINEM ORTHODIPHENOL, EINEM MANGAN- ODER ZINKSALZ, WASSERSTOFFPEROXID, (BI)CARBONAT, EINEM ALKALISCHEN MITTEL UND EINEM TITAN- ODER SCANDIUMSALZ

PROCÉDÉ DE TEINTURE DES CHEVEUX UTILISANT AU MOINS UN ORTHO-DIPHÉNOL, UN SEL DE MANGANÈSE OU DE ZINC, DU PEROXYDE D'HYDROGÈNE, DU (BI) CARBONATE, UN AGENT ALCALIN ET UN SEL DE TITANE OU DE SCANDIUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**23.06.2011 FR 1155523**
**23.06.2011 FR 1155524**
**26.09.2011 US 201161539107 P**
**26.09.2011 US 201161539110 P**

(43) Date of publication of application:
**30.04.2014 Bulletin 2014/18**

(73) Proprietor: **L'Oréal**
**75008 Paris (FR)**

(72) Inventors:
- **CHOISY, Patrick**
**F-37270 Montlouis Sur Loire (FR)**
- **RONDOT, Christophe**
**F-77290 Mitry Mory (FR)**

(74) Representative: **Martin-Charbonneau, Virginie**
**Casalonga & Partners**
**Bayerstrasse 71/73**
**80335 München (DE)**

(56) References cited:
**EP-A2- 2 196 182** **WO-A2-2011/000892**
**FR-A1- 2 951 374**

- **DWECK A C: "Natural ingredients for colouring and styling", INTERNATIONAL JOURNAL OF COSMETIC SCIENCE, KLUWER ACADEMIC PUBLISHERS, DORDRECHT, NL, vol. 24, 1 January 2002 (2002-01-01), pages 287-302, XP002500910, ISSN: 0142-5463, DOI: 10.1046/J.1467-2494.2002.00148.X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to a process for dyeing keratin fibres, in particular human keratin fibres such as the hair, in which the said fibres are treated using one or more cosmetic compositions comprising a) one or more ortho-diphenol derivatives, b) one or more manganese or zinc salts, c) hydrogen peroxide or one or more systems for generating hydrogen peroxide, d) one or more (bi)carbonates or one or more systems for generating (bi)carbonate(s), e) one or more basifying agents other than the (bi)carbonate(s) and f) one or more salts chosen from titanium salts and scandium salts.

It is known practice to obtain "permanent" colorations with dye compositions containing oxidation dye precursors, which are generally known as oxidation bases, such as ortho- or para-phenylenediamines, ortho- or para-aminophenols and heterocyclic compounds. These oxidation bases are colourless or weakly coloured compounds, which, when combined with oxidizing products, may give rise to coloured compounds by a process of oxidative condensation. It is also known that the shades obtained with these oxidation bases may be varied by combining them with couplers or coloration modifiers, the latter being chosen especially from aromatic meta-diamines, meta-aminophenols, meta-diphenols and certain heterocyclic compounds such as indole compounds. This oxidation dyeing process consists in applying to the keratin fibres bases or a mixture of bases and couplers with hydrogen peroxide ($H_2O_2$ or aqueous hydrogen peroxide solution), as oxidizing agent, in leaving it to diffuse, and then in rinsing the fibres. The colorations resulting therefrom are permanent, strong and resistant to external agents, especially to light, bad weather, washing, perspiration and rubbing.

However, the commercial hair dyes that contain them generally have the drawback of staining clothing, of leading to odour and comfort problems, and of degrading keratin fibres. This is particularly the case with oxidation dyes.

In the field of dyeing, it is also known practice to dye keratin materials such as the hair or the skin using ortho-diphenols in the presence of a metal salt especially of manganese (Mn) and/or zinc (Zn). In particular, patent applications FR 2 814 943, FR 2 814 945, FR 2 814 946 and FR 2 814 947 propose compositions for dyeing the skin or keratin fibres, comprising a dye precursor that contains at least one ortho-diphenol, Mn and/or Zn oxides and salts, alkaline agents of hydrogen carbonate type in a particular Mn, Zn/hydrogen carbonate ratio and optionally an enzyme. Patent application EP 2 196 182 discloses also a process for dyeing keratin fibres using at least one ortho-diphenol derivative and at least one metallic salt, particularly chosen from Mn and/or Zn salts, in presence of different alkaline agents including carbonates. According to these documents, it is possible to obtain colorations on keratin materials with atmospheric oxygen or any oxygen-generating system.

The metal salts can also be used as catalysts as disclosed in the patent application WO 2011/000892, This document describes a process for dyeing keratin fibres, and more particularly wools and fabrics, using ortho-diphenol derivatives in presence of a catalyst, such as titanium salts.

However, the colorations obtained are not strong enough and are sparingly chromatic, especially in the case of hair fibres.

Moreover, it is known practice to use metals at acidic pH for dyeing keratin fibres in amounts similar to those employed for dyes using a mordanting process, which consists in preparing the fibres before performing the dyeing operation in order to obtain fast shades (Ullmann's Encyclopaedia "Metal and Dyes", 2005 § 5.1, p. 8).

In particular, patent application FR 2 951 374 describes a process for dyeing keratin fibres using a mordant, preferentially iron sulfate. According to this document, the mordanting composition can be applied on the hair before or after application of the dyeing composition comprising at least one anthrone derivative.

However, this process generally has the drawback of not always respecting the cosmeticity of the hair fibre. Furthermore, the metals used, such as iron, lead to neutral and sparingly chromatic shades. In general, such metals do not make it possible to obtain chromatic shades.

There is thus a real need to develop dyeing processes for obtaining strong, fast and/or chromatic colorations using ortho-diphenols, especially using natural extracts that are rich in ortho-diphenols and less aggressive to keratin fibres. In particular, there is a need to obtain colorations that satisfactorily withstand external agents (light, bad weather or shampooing), which are fast and homogeneous, i.e. showing little coloration selectivity between the root and the end, while at the same time remaining strong and/or chromatic.

This aim is achieved by the present invention, one subject of which is a process for dyeing keratin fibres, in particular human keratin fibres such as the hair, in which the said fibres are treated, in one or more steps, with one or more cosmetic compositions containing, taken together or separately in the said composition(s), the following ingredients:

a) one or more ortho-diphenol derivatives,
b) one or more manganese salts or one or more zinc salts,
c) hydrogen peroxide or one or more systems that generate hydrogen peroxide,
d) one or more (bi)carbonates or one or more systems that generate (bi)carbonate(s),
e) one or more basifying agents other than the (bi)carbonate(s), and
f)one or more salts chosen from titanium salts and scandium salts;

it being understood that the pH of at least one of the compositions comprising at least one of the ingredients a), b), d), e) and/or f) is alkaline, i.e. greater than 7, and that the composition comprising the salt(s) chosen from titanium salts and scandium salts is applied to the keratin fibres as a post-treatment, i.e. as the last step of the dyeing process.

Thus, the keratin fibres are treated at the end of the dyeing process with a composition comprising the titanium salt(s) or the scandium salt(s).

In particular, the cosmetic composition comprising one or more titanium salts or one or more scandium salts is applied to the keratin fibres as a post-treatment.

According to one particular embodiment of the dyeing process according to the invention, compound(s) d) and e) are:

- either in a mixture with ingredients a), b) and c),

  - - or applied separately after application of a cosmetic composition comprising compound(s) a), b) and c),
  - - or applied together with compound c) after application of a cosmetic composition comprising ingredients a) and b).

According to one particular embodiment of the dyeing process of the invention, the salt(s) chosen from titanium salts and scandium salts are applied separately after application of ingredients a) to e).

A subject of the present invention is also a cosmetic composition for dyeing keratin fibres, comprising:

a) one or more ortho-diphenol derivatives,
b) one or more manganese salts or one or more zinc salts,
c) hydrogen peroxide or one or more systems that generate hydrogen peroxide,
d) one or more (bi)carbonates or one or more systems that generate (bi)carbonate(s),
e) one or more basifying agents other than the (bi)carbonate(s), and
f) one or more salts chosen from titanium salts and scandium salts;

it being understood that the pH of the composition is alkaline, i.e. greater than 7 and preferably between 8 and 12. It is particularly between 8 and 10.5.

The dye composition is preferably aqueous.

Another subject of the present invention concerns a multi-compartment device comprising:

a) one or more ortho-diphenol derivatives,
b) one or more manganese salts or one or more zinc salts,
c) hydrogen peroxide or one or more systems that generate hydrogen peroxide,
d) one or more (bi)carbonates or one or more systems that generate (bi)carbonate(s),
e) one or more basifying agents other than the (bi)carbonate(s), and
f) one or more salts chosen from titanium salts and scandium salts;

it being understood that the pH of at least one of the compositions comprising a), b), d), e) and/or f) is alkaline, i.e. greater than 7, and preferably between 8 and 12. It is particularly between 8 and 10.5.

The multi-compartment device or "kit" is suitable for performing the dyeing process according to the invention.

The process according to the invention has the advantage of dyeing human keratin fibres, with fast and surprisingly chromatic dyeing results. In particular, the dyeing process according to the invention leads to colorations that are resistant to washing, perspiration, sebum and light, without impairing the fibres. Furthermore, the dyeing process performed induces satisfactory "build-up" of the coloration.

Thus, another subject of the invention is the use of one or more titanium salts for improving the chromaticity and/or colour build-up of keratin fibres, in particular of human keratin fibres such as the hair, dyed with one or more ortho-diphenol derivatives.

Other subjects, characteristics, aspects and advantages of the invention will emerge even more clearly on reading the description and the examples that follow.

a) <u>ortho-Diphenol derivative</u>

In accordance with the present invention, the dyeing process uses one or more ortho-diphenol derivatives.

The ortho-diphenol derivative(s) may be present in one or more cosmetic compositions used in the course of the dyeing process.

One particular embodiment of the invention concerns ortho-diphenol derivatives or mixtures of compounds

comprising one or more aromatic rings, preferably a benzene ring, comprising at least two hydroxyl groups (OH) borne by two adjacent carbon atoms of the aromatic ring. Particularly, the ortho-diphenol derivative(s) according to the invention are not self-oxidizable derivatives bearing an indole unit. More particularly, they are not 5,6-dihydroxyindole.

The aromatic ring may more particularly be a fused aryl or fused heteroaromatic ring, i.e. optionally containing one or more heteroatoms, such as benzene, naphthalene, tetrahydronaphthalene, indane, indene, anthracene, phenanthrene, isoindole, indoline, isoindoline, benzofuran, dihydrobenzofuran, chroman, isochroman, chromene, isochromene, quinoline, tetrahydroquinoline and isoquinoline, the said aromatic ring comprising at least two hydroxyl groups borne by two adjacent carbon atoms of the aromatic ring. Preferentially, the aromatic ring of the ortho-diphenol derivatives according to the invention is a benzene ring.

The term "fused ring" means that at least two saturated or unsaturated, heterocyclic or non-heterocyclic rings have a common bond, i.e. at least one ring is fused to another ring.

The ortho-diphenols according to the invention may or may not be salified. They may also be in aglycone form (without attached sugar) or in the form of glycosylated compounds.

More particularly, the ortho-diphenol derivative a) represents a compound of formula (I), or an oligomer thereof, in salified or non-salified form:

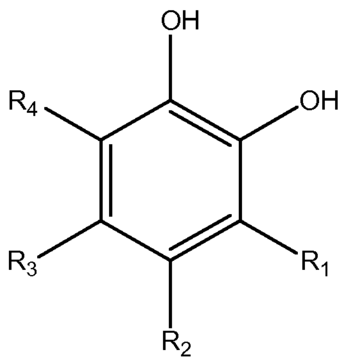

(I)

in which formula (I) the substituents:

- $R_1$ to $R_4$, which may be identical or different, represent:
  - a hydrogen atom;
  - a halogen atom,
  - a hydroxyl radical,
  - a carboxyl radical,
  - an alkyl carboxylate or alkoxycarbonyl radical,
  - an optionally substituted amino radical,
  - an optionally substituted linear or branched alkyl radical,
  - an optionally substituted linear or branched alkenyl radical,
  - an optionally substituted cycloalkyl radical,
  - an alkoxy radical,
  - an alkoxyalkyl radical,
  - an alkoxyaryl radical, the aryl group possibly being optionally substituted,
  - an aryl radical,
  - a substituted aryl radical,
  - a saturated or unsaturated heterocyclic radical, optionally bearing a cationic or anionic charge, optionally substituted and/or optionally fused with an aromatic ring, preferably a benzene ring, the said aromatic ring being optionally substituted particularly with one or more hydroxyl or glycosyloxy groups,
  - a radical containing one or more silicon atoms,

    - in which two of the substituents borne by two adjacent carbon atoms $R_1$ - $R_2$, $R_2$ - $R_3$ or $R_3$ - $R_4$ form, together with the carbon atoms that bear them, a saturated or unsaturated, aromatic or non-aromatic ring, optionally containing one or more heteroatoms and optionally fused with one or more saturated or unsaturated rings optionally containing one or more heteroatoms. Particularly, $R_1$ to $R_4$ together form from one to four rings.

One particular embodiment of the invention concerns ortho-diphenol derivatives of formula (I), of which two adjacent substituents $R_1$ - $R_2$, $R_2$ - $R_3$ or $R_3$ - $R_4$ cannot form with the carbon atoms that bear them a pyrrolyl radical. More particularly, $R_2$ and $R_3$ cannot form a pyrrolyl radical fused to the benzene ring bearing the two hydroxyl groups.
For the purposes of the present invention, and unless otherwise indicated:

The saturated or unsaturated, optionally fused rings may also be optionally substituted.

The alkyl radicals are linear or branched, saturated hydrocarbon-based radicals, generally of $C_1$-$C_{20}$, particularly of $C_1$-$C_{10}$, preferably $C_1$-$C_6$ alkyl radicals, such as methyl, ethyl, propyl, butyl, pentyl and hexyl.
The alkenyl radicals are linear or branched, unsaturated $C_2$-$C_{20}$ hydrocarbon-based radicals; preferably comprising at least one double bond, such as ethylene, propylene, butylene, pentylene, 2-methylpropylene and decylene.
The aryl radicals are fused or nonfused monocyclic or polycyclic carbon-based radicals, preferentially comprising from 6 to 30 carbon atoms, and of which at least one ring is aromatic; the aryl radical is preferentially chosen from phenyl, biphenyl, naphthyl, indenyl, anthracenyl and tetrahydronaphthyl.
The alkoxy radicals are alkyl-oxy radicals with alkyl as defined previously, preferably of $C_1$-$C_{10}$ such as methoxy, ethoxy, propoxy and butoxy.
The alkoxyalkyl radicals are preferably ($C_1$-$C_{20}$)alkoxy($C_1$-$C_{20}$)alkyl radicals, such as methoxymethyl, ethoxymethyl, methoxyethyl, ethoxyethyl, etc.
The cycloalkyl radicals are generally $C_4$-$C_8$ cycloalkyl radicals, preferably cyclopentyl and cyclohexyl radicals. The cycloalkyl radicals may be substituted cycloalkyl radicals, in particular substituted with alkyl, alkoxy, carboxylic acid, hydroxyl, amine and ketone groups.
The alkyl or alkenyl radicals, when they are optionally substituted, may be substituted with at least one substituent borne by at least one carbon atom, chosen from:

- a halogen atom;
- a hydroxyl group;
- a $C_1$-$C_2$ alkoxy radical;
- a $C_1$-$C_{10}$ alkoxycarbonyl radical;
- a $C_2$-$C_4$ (poly)hydroxyalkoxy radical;
- an amino radical;
- a 5- or 6-membered heterocycloalkyl radical;
- an optionally cationic 5- or 6-membered heteroaryl radical, preferentially imidazolium, optionally substituted with a ($C_1$-$C_4$)alkyl radical, preferentially methyl;
- an amino radical substituted with one or two identical or different $C_1$-$C_6$ alkyl radicals, optionally bearing at least:

  * one hydroxyl group,
  * one amino group optionally substituted with one or two optionally substituted $C_1$-$C_3$ alkyl radicals, the said alkyl radicals possibly forming with the nitrogen atom to which they are attached a saturated or unsaturated, optionally substituted 5- to 7-membered heterocycle, optionally comprising at least one other heteroatom identical to or different from nitrogen,
  * a quaternary ammonium group $-N^+R'R''R'''$, $M^-$ for which R', R'' and R''', which may be identical or different, represent a hydrogen atom or a $C_1$-$C_4$ alkyl group; and $M^-$ represents the counterion of the organic or mineral acid or of the corresponding halide;
  * or an optionally cationic 5- or 6-membered heteroaryl radical, preferentially imidazolium, optionally substituted with a ($C_1$-$C_4$)alkyl radical, preferentially methyl;

- an acylamino radical (-NR-COR') in which the radical R is a hydrogen atom, a $C_1$-$C_4$ alkyl radical optionally bearing at least one hydroxyl group and the radical R' is a $C_1$-$C_2$ alkyl radical; a carbamoyl radical ($(R)_2N$-CO-) in which the radicals R, which may be identical or different, represent a hydrogen atom, a $C_1$-$C_4$ alkyl radical optionally bearing at least one hydroxyl group; an alkylsulfonylamino radical ($R'SO_2$-NR-) in which the radical R represents a hydrogen atom, a $C_1$-$C_4$ alkyl radical optionally bearing at least one hydroxyl group and the radical R' represents a $C_1$-$C_4$ alkyl radical or a phenyl radical; an aminosulfonyl radical ($(R)_2N$-$SO_2$-) in which the radicals R, which may be identical or different, represent a hydrogen atom or a $C_1$-$C_4$ alkyl radical optionally bearing at least one hydroxyl group;
- a carboxylic radical in acid or salified (preferably with an alkali metal or a substituted or unsubstituted ammonium) form;
- a cyano group;
- a nitro group;
- a carboxyl or glycosylcarbonyl group;
- a phenylcarbonyloxy group optionally substituted with one or more hydroxyl groups;

- a glycosyloxy group; and
- a phenyl group optionally substituted with one or more hydroxyl groups.

The aryl or heterocyclic radicals or the aryl or heterocyclic part of the radicals, when they are optionally substituted, may be substituted with at least one substituent borne by at least one carbon atom, chosen from:

- a $C_1$-$C_{10}$ and preferably $C_1$-$C_8$ alkyl radical optionally substituted with one or more radicals chosen from hydroxyl, $C_1$-$C_2$ alkoxy, $C_2$-$C_4$ (poly)hydroxyalkoxy, acylamino, amino substituted with two $C_1$-$C_4$ alkyl radicals, which may be identical or different, optionally bearing at least one hydroxyl group, or the two radicals possibly forming, with the nitrogen atom to which they are attached, a saturated or unsaturated, optionally substituted 5- to 7-membered and preferably 5- or 6-membered heterocycle optionally comprising another heteroatom identical to or different from nitrogen;
- a halogen atom;
- a hydroxyl group;
- a $C_1$-$C_2$ alkoxy radical;
- a $C_1$-$C_{10}$ alkoxycarbonyl radical;
- a $C_2$-$C_4$ (poly)hydroxyalkoxy radical;
- an amino radical;
- a 5- or 6-membered heterocycloalkyl radical;
- an optionally cationic 5- or 6-membered heteroaryl radical, preferentially imidazolium, optionally substituted with a ($C_1$-$C_4$)alkyl radical, preferentially methyl;
- an amino radical substituted with one or two identical or different $C_1$-$C_6$ alkyl radicals, optionally bearing at least:

  * one hydroxyl group,
  * one amino group optionally substituted with one or two optionally substituted $C_1$-$C_3$ alkyl radicals, the said alkyl radicals possibly forming with the nitrogen atom to which they are attached a saturated or unsaturated, optionally substituted 5- to 7-membered heterocycle, optionally comprising at least one other heteroatom identical to or different from nitrogen,
  * a quaternary ammonium group $-N^+R'R''R'''$, $M^-$ for which R', R" and R"', which may be identical or different, represent a hydrogen atom or a $C_1$-$C_4$ alkyl group; and $M^-$ represents the counterion of the organic or mineral acid or of the corresponding halide;
  * or an optionally cationic 5- or 6-membered heteroaryl radical, preferentially imidazolium, optionally substituted with a ($C_1$-$C_4$)alkyl radical, preferentially methyl;

- an acylamino radical (-NR-COR') in which the radical R is a hydrogen atom, a $C_1$-$C_4$ alkyl radical optionally bearing at least one hydroxyl group and the radical R' is a $C_1$-$C_2$ alkyl radical; a carbamoyl radical ($(R)_2N$-CO-) in which the radicals R, which may be identical or different, represent a hydrogen atom, a $C_1$-$C_4$ alkyl radical optionally bearing at least one hydroxyl group; an alkylsulfonylamino radical ($R'SO_2$-NR-) in which the radical R represents a hydrogen atom, a $C_1$-$C_4$ alkyl radical optionally bearing at least one hydroxyl group and the radical R' represents a $C_1$-$C_4$ alkyl radical or a phenyl radical; an aminosulfonyl radical ($(R)_2N$-$SO_2$-) in which the radicals R, which may be identical or different, represent a hydrogen atom or a $C_1$-$C_4$ alkyl radical optionally bearing at least one hydroxyl group;
- a carboxylic radical in acid or salified form (preferably with an alkali metal or a substituted or unsubstituted ammonium);
- a cyano group;
- a nitro group;
- a polyhaloalkyl group, preferentially trifluoromethyl;
- a carboxyl or glycosylcarbonyl group;
- a phenylcarbonyloxy group optionally substituted with one or more hydroxyl groups;
- a glycosyloxy group; and
- a phenyl group optionally substituted with one or more hydroxyl groups.

For the purposes of the present invention, the term "glycosyl radical" means a radical derived from a monosaccharide or polysaccharide.

The radicals containing one or more silicon atoms are preferably polydimethylsiloxane, polydiphenylsiloxane, polydimethylphenylsiloxane or stearoxydimethicone radicals.

The heterocyclic radicals are generally radicals comprising in at least one ring one or more heteroatoms chosen from O, N and S, preferably O or N, optionally substituted especially with one or more alkyl, alkoxy, carboxylic acid, hydroxyl, amine or ketone groups. These rings may contain one or more oxo groups on the carbon atoms of the heterocycle.

Among the heterocyclic radicals that may be used, mention may be made of furyl, pyranyl, pyrrolyl, imidazolyl, pyrazolyl, pyridyl and thienyl groups.

More preferably, the heterocyclic groups are fused groups such as benzofuryl, chromenyl, xanthenyl, indolyl, isoindolyl, quinolyl, isoquinolyl, chromanyl, isochromanyl, indolinyl, isoindolinyl, coumarinyl or isocoumarinyl groups, these groups possibly being substituted, in particular with one or more OH groups.

The ortho-diphenols that are useful in the process of the invention may be natural or synthetic. Among the natural ortho-diphenols are compounds that may be present in nature and that are reproduced by chemical (semi)synthesis.

The ortho-diphenol salts of the invention may be salts of acids or bases. The acids may be mineral or organic. Preferably, the acid is hydrochloric acid, which results in chlorides.

The bases may be mineral or organic. In particular, the bases are alkali metal hydroxides such as sodium hydroxide, which leads to sodium salts.

According to one particular embodiment of the invention, the composition comprises as ingredient a) one or more synthetic ortho-diphenol derivatives that do not exist in nature.

According to another preferred embodiment of the invention, the composition that is useful in the process for dyeing keratin fibres comprises as ingredient a) one or more natural ortho-diphenol derivatives.

More particularly, the ortho-diphenols that may be used in the process of the invention according to a) are in particular:

- flavanols, for instance catechin and epicatechin gallate,
- flavonols, such as quercetin,
- anthocyanidins, for instance cyanidin, delphinidin and petunidin,
- anthocyanins or anthocyans, for instance myrtillin,
- ortho-hydroxybenzoates, for example gallic acid salts,
- flavones, such as luteolin,
- hydroxystilbenes, for example tetrahydroxy-3,3’, 4,5’-stilbene, optionally oxylated (for example glucosylated),
- 3,4-dihydroxyphenylalanine and derivatives thereof,
- 2,3-dihydroxyphenylalanine and derivatives thereof,
- 4,5-dihydroxyphenylalanine and derivatives thereof,
- dihydroxycinnamates such as caffeic acid and chlorogenic acid,
- ortho-polyhydroxycoumarins,
- ortho-polyhydroxyisocoumarins,
- ortho-polyhydroxycoumarones,
- ortho-polyhydroxyisocoumarones,
- ortho-polyhydroxychalcones,
- ortho-polyhydroxychromones,
- ortho-polyhydroxyquinones,
- ortho-hydroxyxanthones,
- 1,2 dihydroxybenzene and derivatives thereof,
- 1,2,4-trihydroxybenzene and derivatives thereof,
- 1,2,3-trihydroxybenzene and derivatives thereof,
- 2,4,5-trihydroxytoluene and derivatives thereof,
- proanthocyanidins and especially the proanthocyanidins A1, A2, B1, B2, B3 and C1,
- proathocyanins,
- tannic acid,
- ellagic acid,
- and mixtures of the preceding compounds,

particularly catechin, quercetin, braziline, hematin, hematoxylin, chlorogenic acid, caffeic acid, gallic acid, catechol, gallic acid, L-DOPA, pelargonidin, cyanidin, (-)-epicatechin, (-)-epigallocatechin, (-)-epigallocatechin 3-gallate (EGCG), (+)-catechin, isoquercetin, pomiferin, esculetin, 6,7-dihydroxy-3-(3-hydroxy-2,4-dimethoxyphenyl)coumarin, santalin AC, mangiferin, butein, maritimetin, sulfuretin, robtein, betanidin, pericampylinone A, theaflavin, proanthocyanidin A2, proanthocyanidin B2, proanthocyanidin C1, procyanidins DP 4-8, tannic acid, purpurogallin, 5,6-dihydroxy-2-methyl-1,4-naphthoquinone, alizarin, wedelolactone, variegatic acid, gomphidic acid, xerocomic acid, carnosol, and natural extracts containing them.

The term "carboxylate" means a carboxylic acid salt.

The term "carboxylic acid" means a compound comprising at least one carboxylic acid group-C(O)-OH, preferably between 1 and 4 carboxylic acid groups, such as 1 or 2. More particularly, the carboxylic acid is chosen from: i)

$(C_1-C_{10})$alkyl$[C(O)-OH]_n$ and ii) het-$[C(O)-OH]_n$, with n being an integer between 1 and 4 inclusively and preferably between 1 and 2, het representing a heterocyclic group such as pyrrolidone, the alkyl or het group possibly being optionally substituted with one or more groups, chosen especially from OH and (di)$(C_1-C_6)$(alkyl)amino.

When the dye precursors have D and L forms, the two forms may be used in the compositions according to the invention, as may the racemic mixtures.

Preferentially, the ortho-diphenol(s) according to the invention are chosen from flavanols, flavonols, ortho-hydroxybenzoates, isoflavones and neoflavones.

According to one embodiment, the natural ortho-diphenols are derived from extracts of animals, bacteria, fungi, algae, plants and fruit, used in their entirety or partially. In particular concerning plants, the extracts are obtained from fruit including citrus fruit, legumes, trees and shrubs. Mixtures of these extracts that are rich in ortho-diphenols as defined previously may also be used.

Preferably, the natural ortho-diphenol(s) of the invention are derived from extracts of plants or plant parts.

For the purposes of the invention, these extracts will be assimilated as compound a).

The extracts are obtained by extraction of various plant parts, for instance the root, the wood, the bark, the leaf, the flower, the fruit, the seed, the clove or the peel.

Among the plant extracts, mention may be made of extracts of tea leaves and of rose.

Among the fruit extracts, mention may be made of extracts of apple, of grape (in particular of grape seed) or extracts of cocoa beans and/or pods.

Among the legume extracts, mention may be made of extracts of potato or of onion peel.

Among the extracts of tree wood, mention may be made of extracts of pine bark and extracts of campeachy wood;

Mixtures of plant extracts may also be used.

According to one particular embodiment of the invention, the ortho-diphenol derivative(s) are natural extracts, rich in ortho-diphenols. According to one preferred mode, the ortho-diphenol derivative(s) are solely natural extracts.

Preferentially, the ortho-diphenol(s) according to the invention are chosen from catechin, quercetin, haematin, haematoxylin, brasilin, gallic acid, and natural extracts containing them chosen from grape marc, pine bark, green tea, onion, cocoa bean, logwood, redwood and gall nut.

Even more preferentially, the ortho-diphenol(s) used in one or more compositions according to the invention are chosen from catechin, quercetin, gallic acid and haematoxylin.

The natural extracts according to the invention may be in the form of powders or liquids. Preferably, the extracts of the invention are in the form of powders.

According to the invention, the natural, synthetic ortho-diphenol derivative(s), and/or the natural extract(s) used as ingredient a) in one or more cosmetic compositions that are useful in the process according to the invention preferably represent from 0.001% to 20% by weight relative to the total weight of the composition(s) containing the ortho-diphenol(s) or the extract(s).

As regards the pure ortho-diphenols, the content in the composition(s) containing them is preferably between 0.001% and 5% by weight of each of these compositions.

As regards the extracts, the content in the composition(s) containing the extracts per se is preferably between 0.5% and 20% by weight of each of these compositions.

b) Manganese or zinc salt

The dyeing process according to the invention uses one or more manganese (Mn) salts or one or more zinc (Zn) salts. The manganese or zinc salt(s) may be employed in one or more cosmetic compositions used in the course of the dyeing process.

For the purposes of the present invention, the term "salts" means the oxides of these metals and salts per se derived especially from the action of an acid on a metal.

Preferably, the salts are not oxides.

Among the salts, mention may be made of halides such as chlorides, fluorides and iodides; sulfates, phosphates; nitrates; perchlorates and carboxylic acid salts and polymeric salts, and also mixtures thereof.

More particularly, manganese salt is other than manganese carbonate, manganese hydrogen carbonate or manganese dihydrogen carbonate.

The carboxylic acid salts that may be used in the invention also include salts of hydroxylated carboxylic acids such as gluconate.

As examples of polymeric salts, mention may be made of manganese pyrrolidonecarboxylate.

By way of example, mention may be made of manganese chloride, manganese fluoride, manganese acetate tetrahydrate, manganese lactate trihydrate, manganese phosphate, manganese iodide, manganese nitrate trihydrate, manganese bromide, manganese perchlorate tetrahydrate, manganese sulfate monohydrate and manganese gluconate. The salts advantageously used are manganese gluconate and manganese chloride.

Among the zinc salts, mention may be made of zinc sulfate, zinc gluconate, zinc chloride, zinc lactate, zinc acetate, zinc glycinate and zinc aspartate.

The manganese and zinc salts may be introduced in solid form into the compositions or may be derived from a natural, mineral or spring water that is rich in these ions or alternatively from seawater (especially the Dead Sea). They may also originate from mineral compounds, for instance earths, ochres such as clays (for example green clay) or even from a plant extract containing them (cf. for example patent FR 2 814 943).

According to one preferred embodiment of the invention, the manganese or zinc salt(s) used represent from 0.001% to 0.1% by weight approximately relative to the total weight of the composition(s) containing the said metal salt(s), and even more preferentially from 0.05% to 10% by weight approximately.

Particularly, the metal salts of the invention are in oxidation state 2, such as Mn (II) and Zn (II).

Preferably, the dyeing process of the invention uses a cosmetic composition comprising one or more manganese salts, in particular Mn(II) metal salts.

Even more preferentially, the manganese salt(s) are chosen from manganese carboxylic acid salts, especially manganese gluconate, and manganese halides such as manganese chloride; and more particularly manganese gluconate.

c) Hydrogen peroxide or system that generates hydrogen peroxide

In the context of the present invention, the third constituent is hydrogen peroxide or a system that generates hydrogen peroxide, such as:

a) urea peroxide;
b) polymeric complexes that can release hydrogen peroxide, such as polyvinylpyrrolidone/$H_2O$ in particular in the form of powders, and the other polymeric complexes described in US 5 008 093; US 3 376 110; US 5 183 901;
c) oxidases that produce hydrogen peroxide in the presence of a suitable substrate (for example glucose in the case of glucose oxidase or uric acid with uricase);
d) metal peroxides that generate hydrogen peroxide in water, for instance calcium peroxide or magnesium peroxide;
e) perborates; or
f) percarbonates.

According to one preferred embodiment of the invention, the composition contains one or more systems that generate hydrogen peroxide, chosen from a) urea peroxide, b) polymeric complexes that can release hydrogen peroxide, chosen from polyvinylpyrrolidone/$H_2O_2$; c) oxidases; e) perborates and f) percarbonates.

Particularly, the third constituent is hydrogen peroxide.

Moreover, the composition(s) comprising hydrogen peroxide or the hydrogen peroxide generator may also contain various adjuvants conventionally used in hair dye compositions and as defined hereinbelow.

According to one particular mode of the invention, the hydrogen peroxide used or the system(s) used that generate hydrogen peroxide preferably represent from 0.001% to 12% by weight of hydrogen peroxide relative to the total weight of the composition(s) containing them, and even more preferentially from 0.2% to 2.7% by weight.

d) (Bi)carbonate(s) or system that generates (bi)carbonate(s)

In accordance with the present invention, the dyeing process uses one or more (bi)carbonates or one or more systems that generate (bi)carbonates.

For the purposes of the present invention, the term "system that generates (bi)carbonate" means a system that generates (bi)carbonate in situ, for instance carbon dioxide in water or by buffering carbonate with a mineral or organic acid.

The (bi)carbonates or the system(s) that generate (bi)carbonates may be employed in one or more cosmetic compositions in the course of the dyeing process.

The (bi)carbonate(s) are chosen from:

a) carbonates of alkali metals ($Met_2^+$, $CO_3^{2-}$), of alkaline-earth metals ($Met'^{2+}$, $CO_3^{2-}$) of ammonium ($(R''_4N^+)_2$,$CO_3^{2-}$) or of phosphonium ($(R''_4P+)_2$,$CO_3^{2-}$ with Met' representing an alkaline-earth metal and Met representing an alkali metal, and R", which may be identical or different, represent a hydrogen atom, an optionally substituted group ($C_1$-$C_6$)alkyl such as hydroxyethyl),
b) bicarbonates, also known as hydrogen carbonates, of the following formulae:

➢ $R'^+$, $HCO_3^-$ with R' representing a hydrogen atom, an alkali metal, an ammonium group $R''_4N^+$- or a phos-

phonium group $R''_4P^+$- in which R", which may be identical or different, represent a hydrogen atom, an optionally substituted group ($C_1$-$C_6$)alkyl such as hydroxyethyl and, when R' represents a hydrogen atom, the hydrogen carbonate is then known as a dihydrogen carbonate ($CO_2$, $H_2O$); and

➢ $Met'^{2+}$ $(HCO_3^-)_2$ with Met' representing an alkaline-earth metal.

More particularly, the (bi)carbonate(s) are chosen from alkali metal, alkaline-earth metal or ammonium (bi)carbonates; preferentially alkali metal or ammonium (bi)carbonates.

Mention may be made of Na, K, Mg and Ca carbonates or hydrogen carbonates and mixtures thereof, and in particular sodium hydrogen carbonate. These hydrogen carbonates may originate from a natural water, for example spring water from the Vichy basin or from La Roche Posay or Badoit water (cf. for example, patent FR 2 814 943). Particularly, mention may be made of sodium carbonate [497-19-8] = $Na_2CO_3$, sodium hydrogen carbonate or sodium bicarbonate [144-55-8] = $NaHCO_3$, and sodium dihydrogen carbonate = $Na(HCO_3)_2$.

According to the invention, the (bi)carbonate(s) used preferably represent from 0.001% to 10% by weight relative to the total weight of composition(s) containing the (bi)carbonate(s), and even more preferentially from 0.005% to 5% by weight.

Preferably, the (bi)carbonate used in the composition during the dyeing process is ammonium (bi)carbonate or sodium bicarbonate.

e) Basifying agents other than the (bi)carbonate(s)

The basifying agent used in the dyeing process according to the invention as fifth ingredient is other than the (bi)carbonate(s) iv) as defined previously. This is an agent for increasing the pH of the composition(s) in which it is present. The basifying agent is a Bronsted, Lowry or Lewis base. It may be mineral or organic.

Particularly, the said agent is chosen from i) aqueous ammonia, ii) alkanolamines such as monoethanolamine, diethanolamine, triethanolamine and derivatives thereof, iii) oxyethylenated and/or oxypropylenated ethylenediamines, iv) mineral or organic hydroxides, v) alkali metal silicates such as sodium metasilicate, vi), amino acids, preferably basic amino acids such as arginine, lysine, ornithine, citrulline and histidine, and vii) the compounds of formula (II) below:

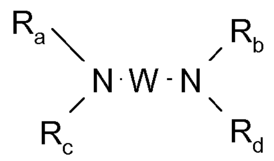

(II)

in which:

- W is a divalent ($C_1$-$C_8$)alkylene group, preferably propylene optionally substituted especially with a hydroxyl group or a $C_1$-$C_4$ alkyl radical;
- $R_a$, $R_b$, $R_c$ and $R_d$, which may be identical or different,

represent a hydrogen atom or a $C_1$-$C_4$ alkyl or $C_1$-$C_4$ hydroxyalkyl radical.

The mineral or organic hydroxides are preferably chosen from i) hydroxides of an alkali metal, ii) hydroxides of an alkaline-earth metal, for instance sodium hydroxide or potassium hydroxide, iii) hydroxides of a transition metal, such as hydroxides of metals from groups III, IV, V and VI, iv) hydroxides of lanthanides or actinides, quaternary ammonium hydroxides and guanidinium hydroxide.

Preferentially, whether it is for the composition according to the invention or the process used in the invention, the basifying agent is not sodium hydroxide NaOH. More generally and preferentially, whether it is for the composition according to the invention or the process used in the invention, the basifying agent is not an alkali metal hydroxide XOH with X = alkali metal.

The hydroxide may be formed *in situ*, for instance guanidine hydroxide, by reacting calcium hydroxide and guanidine carbonate.

The basifying agent(s) d) as defined previously preferably represent from 0.001% to 10% by weight relative to the weight of the composition(s) containing them, and more particularly from 0.005% to 8% by weight of the composition.

Preferably, the alkaline agent(s) are chosen from alkanolamines, in particular monoethanolamine.

f) Salts

According to one preferred embodiment, the ingredient f) used in accordance with the present invention is a titanium salt.

- Titanium salts:

For the purposes of the present invention, the term "titanium salt" means the oxides of this metal and salts per se derived especially from the action of an acid on a metal.

According to one particular embodiment of the invention, the titanium derivative(s) are chosen from:

i) titanium salts of oxidation state 4 such as potassium titanium oxalate dihydrate $K_2TiO(C_2O_4)_2$-$2H_2O$, $Ti(SO_4)_2$,
ii) titanium oxides chosen especially from titanium oxides and salts thereof, hydrates thereof and supported forms thereof,
iii) titanium complexes such as the phthalocyanins described, for example, in document US 3 931 249.

Among the titanium oxides, examples that may be mentioned include the titanium oxides and hydroxides described in Ullhamnn encyclopedia "Titanium, Titanium Alloys and Titanium Compounds", 2005 Wiley-VCH Verlag GmbH & Co. KGaA, Weinheim, 10.1002/14356007.a27 095, pp. 1-33. More particularly, the metal derivative(s) are titanium (Ti) and are chosen from titanium (III) hydroxide and oxide $Ti(OH)_3$ and $TiO_3$, dititanium trioxide $Ti_2O_3$, alkaline-earth metal titanium trioxides, alkaline-earth metal titanium pentoxides, the titanates of general formula $MIITiO_4$ in which MII represents a metal Mg, Zn, Mn or Co, peroxytitanic acid and the peroxytitanates $H_4TiO_5$, titanium (II) dioxide $TiO_2$, titanium disulfide $TiS_2$.

The titanium oxides may also originate from minerals such as anatase and rutile that contain $TiO_2$; perovskite containing calcium trioxide $CaTiO_3$, sphene or titanite containing $CaTi(SiO_4)O$.

Preferentially, the titanium salts are chosen from salts of titanium of oxidation state 4 such as potassium titanium oxalate dihydrate $K_2TiO(C_2O_4)_2$-$2H_2O$.

According to another preferred embodiment, the ingredient f) in accordance with the present invention is a scandium salt.

- Scandium salts:

For the purposes of the present invention, the term "scandium salt" means the oxides of this metal and salts per se derived especially from the action of an acid on a metal.

Preferably, the scandium salts are not oxides.

Among the scandium salts, mention may be made of hydroxide, halides such as the chloride, sulfate, nitrate, carboxylic acid salts such as acetate and gluconate, and sulfonic acid salts, in particular (poly)halo($C_1$-$C_6$)alkylsulfonates such as trifluoromethanesulfonate.

By way of example, mention may be made of scandium hydroxide, scandium chloride, scandium sulfate, scandium nitrate, anhydrous or hydrated scandium acetate, and scandium trifluoromethanesulfonate.

Preferably, the scandium salt used in the dyeing process is anhydrous or hydrated scandium acetate.

In one preferred embodiment of the invention, the amount in moles of titanium or scandium salts used in the process or in the composition is equivalent in moles to that of the dyes.

The titanium or scandium salt(s) are present in the cosmetic composition(s) used in the process according to the invention in a content ranging from 0.001% to 20% by weight relative to the total weight of the composition(s) containing them.

Preferentially, the titanium or scandium salts according to the invention are soluble in water to a proportion of at least 0.0001 g/L.

The titanium or scandium salts may be introduced in solid form into the compositions or may be derived from a natural, mineral or spring water that is rich in these ions or alternatively from seawater (especially the Dead Sea). They may also originate from mineral compounds, for instance earths, ochres such as clays (for example green clay) or even from a plant extract containing them (cf. for example patent FR 2 814 943).

- Water:

According to one embodiment of the invention, water is preferably included in the process of the invention. It may originate from the moistening of the keratin fibres and/or from the composition(s) comprising compounds a) to f) as defined previously or from one or more other compositions.

Preferably, the water comes from at least one composition comprising at least one compound chosen from a) to f) as defined previously.

- <u>Cosmetic compositions:</u>

The cosmetic compositions according to the invention are cosmetically acceptable, i.e. they comprise a dye support that generally contains water or a mixture of water and of one or more organic solvents or a mixture of organic solvents.

The term "organic solvent" means an organic substance that is capable of dissolving or dispersing another substance without chemically modifying it.

- <u>Organic solvents:</u>

Examples of organic solvents that may be mentioned include $C_1$-$C_4$ lower alcohols, such as ethanol and isopropanol; polyols and polyol ethers such as 2-butoxyethanol, propylene glycol, propylene glycol monomethyl ether, diethylene glycol monoethyl ether and monomethyl ether, hexylene glycol, and also aromatic alcohols, for instance benzyl alcohol or phenoxyethanol.

The organic solvents are present in proportions preferably of between 1% and 40% by weight approximately and more preferably still between 5% and 30% by weight approximately relative to the total weight of the dye composition.

- <u>Adjuvants:</u>

The composition(s) of the dyeing process in accordance with the invention may also contain various adjuvants conventionally used in hair dye compositions, such as anionic, cationic, nonionic, amphoteric or zwitterionic surfactants or mixtures thereof, anionic, cationic, nonionic, amphoteric or zwitterionic polymers or mixtures thereof, mineral or organic thickeners, and in particular anionic, cationic, nonionic and amphoteric polymeric associative thickeners, antioxidants, penetrants, sequestrants, fragrances, buffers, dispersants, conditioning agents, for instance volatile or non-volatile, modified or unmodified silicones, film-forming agents, ceramides, preserving agents and opacifiers.

The said adjuvants are preferably chosen from surfactants such as anionic or nonionic surfactants or mixtures thereof and mineral or organic thickeners.

The above adjuvants are generally present in an amount for each of them of between 0.01% and 40% by weight relative to the weight of the composition, and preferably between 0.1% and 20% by weight relative to the weight of the composition.

Needless to say, a person skilled in the art will take care to select this or these additional compound(s) such that the advantageous properties intrinsically associated with the composition(s) that are useful in the dyeing process in accordance with the invention are not, or are not substantially, adversely affected by the envisaged addition(s).

- <u>Additional dyes:</u>

The dyeing process using ingredients a) to f) as defined previously may also use one or more additional direct dyes. These direct dyes are chosen, for example, from those conventionally used in direct dyeing, and among which mention may be made of any commonly used aromatic and/or non-aromatic dye such as neutral, acidic or cationic nitrobenzene direct dyes, neutral, acidic or cationic azo direct dyes, natural direct dyes other than ortho-diphenols, neutral, acidic or cationic quinone and in particular anthraquinone direct dyes, azine, triarylmethane, indoamine, methine, styryl, porphyrin, metalloporphyrin, phthalocyanin and methine cyanine direct dyes, and fluorescent dyes.

Among the natural direct dyes, mention may be made of lawsone, juglone, indigo, isatin, curcumin, spinulosin, apigenidin and orceins. Extracts or decoctions containing these natural dyes and in particular henna-based poultices or extracts, may also be used.

According to the invention, the direct dye(s) used in the composition according to the invention which comprises ingredients a) to f) as defined previously, or the composition(s) of the dyeing process according to the invention, preferably represent from 0.001% to 10% by weight approximately and even more preferentially from 0.05% to 5% by weight approximately relative to the total weight of the composition(s).

The composition according to the invention or the composition(s) of the process using ingredients a) to f) as defined previously may also comprise one or more oxidation bases and/or one or more couplers conventionally used for the dyeing of keratin fibres.

Among the oxidation bases, mention may be made of para-phenylenediamines, bis(phenyl)alkylenediamines, para-aminophenols, bis-para-aminophenols, ortho-aminophenols and heterocyclic bases, and the addition salts thereof.

Among these couplers, mention may be made especially of meta-phenylenediamines, meta-aminophenols,

meta-diphenols, naphthalene-based couplers and heterocyclic couplers, and the addition salts thereof.

The said base(s) present in the said composition(s) used in the process are each generally present in an amount of between 0.001% and 10% by weight relative to the total weight of the composition(s) containing them.

The cosmetic composition(s) of the invention may be in various galenical forms, such as a powder, a lotion, a mousse, a cream or a gel, or in any other form that is suitable for dyeing keratin fibres. They may also be packaged in a propellant-free pump-action bottle or under pressure in an aerosol container in the presence of a propellant and form a foam.

- pH of the composition(s):

In accordance with the present invention, the pH of at least one of the cosmetic compositions comprising at least one of the ingredients a), b), d), e) and/or f) is alkaline, i.e. greater than 7, preferably between 8 and 12 and more preferably between 8 and 10.

In other words, at least one of the cosmetic compositions used in the dyeing process of the invention has an alkaline pH, i.e. greater than 7.

According to one embodiment, the pH of the cosmetic composition(s) containing d) one or more (bi)carbonate(s) or one or more systems that generate (bi)carbonate(s) is alkaline, i.e. greater than 7, preferably between 8 and 12 and more particularly between 8 and 10.

Preferably, the (bi)carbonate(s) or the system(s) that generate(s) (bi)carbonate(s) are used in a single cosmetic composition and the pH of the said composition is alkaline, i.e. greater than 7, preferably between 8 and 12 and more particularly between 8 and 10.

According to one particular embodiment, the pH of the cosmetic composition(s) comprising d) one or more (bi)carbonate(s) or one or more systems that generate (bi)carbonate(s) and e) one or more basifying agents other than the (bi)carbonate(s) is alkaline, i.e. greater than 7, preferably between 8 and 12 and more particularly between 8 and 10.

In other words, preferably, the (bi)carbonate(s) or the system(s) that generate(s) (bi)carbonate(s) d) and the basifying agent(s) other than the (bi)carbonate(s) e) are used in a single cosmetic composition and the pH of the said composition is alkaline, i.e. greater than 7, preferably between 8 and 12, more particularly between 8 and 10 and especially between 9 and 10.

Even more preferentially, the (bi)carbonate(s) chosen from ammonium bicarbonates and the basifying agents chosen from alkanolamines are used in a single cosmetic composition and the pH of the said composition is alkaline, i.e. greater than 7, preferably between 8 and 12, more particularly between 8 and 10 and especially between 9 and 10.

According to one embodiment, if one or more cosmetic compositions used in the dyeing process do not contain (bi)carbonates, the pH of the composition(s) containing hydrogen peroxide or a system that generates hydrogen peroxide is preferably less than 7 and more particularly between 1 and 5.

Preferably, the composition(s) containing the ortho-diphenol(s) and not containing any (bi)carbonates have a pH of less than 7 and preferably between 3 and 6.5.

According to one particular embodiment of the invention, the cosmetic composition(s) containing the manganese or zinc salt(s) b) and not containing any (bi)carbonates have a pH of less than 7 and preferably between 3 and 6.5.

According to one particular embodiment of the invention, the cosmetic composition(s) containing the titanium salt(s) or the scandium salts f) and not containing any (bi)carbonates have a pH of less than 7 and preferably between 3 and 6.5.

The pH of these compositions may be adjusted to the desired value by means of basifying agents as defined previously in d) or by using acidifying agents usually used in the dyeing of keratin fibres, or alternatively by means of standard buffer systems.

Among the acidifying agents for the compositions used in the invention, examples that may be mentioned include mineral or organic acids, for instance hydrochloric acid, orthophosphoric acid or sulfuric acid, carboxylic acids, for instance acetic acid, tartaric acid, citric acid and lactic acid, and sulfonic acids.

Among the basifying agents, they are agents as defined previously under the heading *"d) basifying agent(s)"*.

- Dyeing process in one or more steps

According to one embodiment, the process for dyeing keratin fibres is performed in a single step by applying to the keratin fibres an aqueous dye composition comprising:

a) one or more ortho-diphenol derivatives,
b) one or more manganese salts or one or more zinc salts,
c) hydrogen peroxide or one or more systems that generate hydrogen peroxide,
d) one or more (bi)carbonates or one or more systems that generate (bi)carbonate(s),

e) one or more basifying agents other than the (bi)carbonate(s), and
f) one or more salts chosen from titanium salts and scandium salts;

The leave-on time after application is generally set at between 3 and 120 minutes, preferentially between 10 and 60 minutes and more preferentially between 15 and 45 minutes.
In this embodiment, the cosmetic dye composition comprising the ingredients a), b), c), d), e) and f) has an alkaline pH, i.e. greater than 7, preferably between 8 and 12 and more particularly between 8 and 10.
According to another embodiment, the process for dyeing keratin fibres is performed in two or three steps.
According to one particular embodiment of the invention, the process for dyeing keratin fibres is performed in two steps by applying to the keratin fibres a dye composition comprising the ingredients a), b), c), d) and e) as defined previously, and then, in a second step, a composition comprising the ingredients f) as defined previously is applied to the said keratin fibres, it being understood that at least one of the two compositions is aqueous. Preferably, the composition comprising the ortho-diphenol derivative(s) is aqueous. Even more preferentially, the two compositions used in this embodiment are aqueous.
For this process, the leave-on time after application for the first step is generally set at between 3 and 120 minutes, preferentially between 10 and 60 minutes and more preferentially between 15 and 45 minutes. The application time of the composition comprising ingredient f) in the second step is generally set at between 3 and 120 minutes, preferentially between 3 and 60 minutes and more preferentially between 5 and 30 minutes.
According to another embodiment, the process for dyeing keratin fibres is performed in three steps.
In a first variant of the three-step process, the first step consists in applying to the said keratin fibres a composition comprising ingredients a), b) and c) as defined previously, and then, in a second step, a composition comprising ingredients d) and e) as defined previously is applied to the said fibres, and then, in a third step, a composition comprising ingredient f) is applied to the said fibres, it being understood that at least one of the three compositions is aqueous. Preferably, the composition comprising the ortho-diphenol derivative(s) is aqueous. Even more preferentially, the three compositions used in this embodiment are aqueous.
In a second variant of the three-step process, the first step consists in applying to the said keratin fibres a composition comprising ingredients a) and b) as defined previously, and then, in a second step, a composition comprising ingredients c), d) and e) as defined previously is applied to the said fibres, and then, in a third step, a composition comprising ingredient f) is applied to the said fibres, it being understood that at least one of the three compositions is aqueous.
For these last two processes, the leave-on time after application for the first step is generally set at between 3 and 120 minutes, preferentially between 10 and 60 minutes and more preferentially between 15 and 45 minutes. The application time of the composition used in the second step is generally set at between 3 and 120 minutes, preferentially between 3 and 60 minutes and more preferentially between 5 and 30 minutes. The application time of the composition used in the third step is generally set at between 3 and 120 minutes, preferentially between 3 and 60 minutes and more preferentially between 5 and 30 minutes.
According to one embodiment, the process for dyeing keratin fibres is performed in one or more steps by applying to the keratin fibres one or more cosmetic compositions comprising:

a) one or more ortho-diphenol derivatives, especially those chosen from catechin, quercetin, haematoxylin and gallic acid,
b) one or more manganese salts chosen from carboxylic acid salts of manganese, in particular manganese gluconate,
c) hydrogen peroxide,
d) one or more (bi)carbonates, especially ammonium bicarbonates,
e) one or more basifying agents chosen from alkanolamines,
f) one or more titanium salts, in particular salts of titanium of oxidation state 4 such as potassium titanium oxalate dihydrate $K_2TiO(C_2O_4)_2$-$2H_2O$, or one or more scandium salts, in particular carboxylic acid salts of scandium, especially anhydrous or hydrated scandium acetate;

it being understood that the composition comprising ingredient d) has an alkaline pH, i.e. greater than 7, in particular between 8 and 10, and that the composition comprising the salts chosen from titanium salts and scandium salts is applied to the keratin fibres in the last step of the dyeing process.
Preferably, the process for dyeing keratin fibres is performed in three steps.
In particular, the dyeing process is performed in three steps by applying to the keratin fibres:

i) a cosmetic composition comprising a) one or more ortho-diphenol derivatives, b) one or more manganese solvents chosen from carboxylic acid salts of manganese, and c) hydrogen peroxide,
ii) a cosmetic composition comprising d) one or more (bi)carbonates chosen from ammonium bicarbonates and e)

one or more basifying agents chosen from alkanolamines,

iii) a cosmetic composition comprising f) one or more titanium salts, in particular salts of titanium of oxidation state 4 such as potassium titanium oxalate dihydrate $K_2TiO(C_2O_4)_2$-$2H_2O$, or one or more scandium salts, in particular carboxylic acid salts of scandium, especially anhydrous or hydrated scandium acetate,

it being understood that the composition comprising ingredients d) and e) has an alkaline pH, i.e. greater than 7, in particular between 8 and 10, and that the composition comprising the salts chosen from titanium salts and scandium salts is applied to the keratin fibres in the last step of the dyeing process.

More particularly, the ortho-diphenol(s) used in composition i) may be chosen from catechin, quercetin, haematin, haematoxylin, brasilin, gallic acid, and natural extracts containing them chosen from grape marc, pine bark, green tea, onion, cocoa bean, logwood, redwood and gall nut. Even more particularly, the ortho-diphenol(s) are chosen from catechin, quercetin, haematoxylin and gallic acid.

Irrespective of the application method, the application temperature is generally between room temperature (15 to 25°C) and 80°C and more particularly between 15 and 45°C. Thus, after application of the composition according to the invention, the head of hair may advantageously be subjected to a heat treatment by heating to a temperature of between 30 and 60°C. In practice, this operation may be performed using a styling hood, a hairdryer, an infrared ray dispenser or other standard heating appliances.

Use may be made, both as means for heating and smoothing the hair, of a heating iron at a temperature of between 60°C and 220°C and preferably between 120°C and 200°C.

Irrespective of the mode of application, mechanical wiping and/or drying and/or rinsing of the keratin fibres may optionally be performed between each step, especially before performing the step comprising the application of a composition comprising ingredient d) and/or e), especially before performing the final step comprising the application of a composition containing the titanium salt(s) or the scandium salts f).

The steps of intermediate mechanical wiping and drying are also known as "controlled non-rinsing" to distinguish from "standard abundant rinsing with water" and "non-rinsing". The term "mechanical wiping of the fibres" means rubbing an absorbent article on the fibres and physical removal, by means of the absorbent article, of the excess ingredient(s) that have not penetrated into the fibres. The absorbent article may be a piece of fabric such a towel, particularly a terry towel, a cloth or absorbent paper such as household roll towel.

According to one particularly advantageous process of the invention, the mechanical wiping is performed without total drying of the fibre, leaving the fibre moist.

The term "drying" means the action of evaporating the organic solvents and/or water that are in one or more compositions used in the process of the invention, comprising or not comprising one or more ingredients a) to f) as defined previously. The drying may be performed with a source of heat (convection, conduction or radiation) by sending, for example, a stream of hot gas such as air necessary to evaporate the solvent(s). Sources of heat that may be mentioned include a hairdryer, a hairstyling hood, a hair-straightening iron, an infrared ray dispenser or other standard heating appliances.

One particular mode of the invention concerns a dyeing process that is performed at room temperature (25°C).

In all the particular embodiments and variants of the processes described previously, the compositions mentioned are ready-to-use compositions that may result from the extemporaneous mixing of two or more than two compositions and especially of compositions present in dyeing kits.

- Dyeing device or "kit"

Another subject of the invention is a multicompartment dyeing device or "kit". Advantageously, this kit comprises from 2 to 7 compartments containing from 2 to 7 compositions in which are distributed the ingredients a) one or more ortho-diphenol derivatives, b) one or more manganese salts or one or more zinc salts, c) hydrogen peroxide or one or more systems that generate hydrogen peroxide, d) one or more (bi)carbonates or one or more systems that generate (bi)carbonate(s), e) one or more basifying agents other than the (bi)carbonate(s) and f) one or more salts chosen from titanium salts and scandium salts, the said compositions possibly being aqueous or pulverulent, with, particularly, at least one of the said compositions being aqueous.

According to a first variant, the kit comprises seven compartments, the first six compartments comprising, respectively, the powdered ingredients a), b), c), d), e) and f) as defined previously and the sixth compartment containing an aqueous composition such as water. In this case, the compound(s) c) are hydrogen peroxide precursors.

Another variant concerns a six-compartment kit, at least one of which contains an aqueous composition, and in which the other compartments comprise an ingredient a) to f) as defined previously.

In another variant, the device comprises six compartments: a first compartment comprising a composition containing a) one or more ortho-diphenol derivatives, a second compartment comprising b) one or more manganese salts or one or more zinc salts, a third compartment comprising c) hydrogen peroxide or one or more systems that

generate hydrogen peroxide, a fourth compartment containing d) one or more (bi)carbonates or one or more systems that generate (bi)carbonate(s), a fifth compartment containing e) one or more basifying agents other than the (bi)carbonate(s), and a sixth compartment containing f) one or more salts chosen from titanium salts and scandium salts. Preferably, at least one of these compositions is aqueous.

Another preferred embodiment concerns a device comprising four compartments:

(i) a first compartment contains a composition containing:

a) one or more ortho-diphenol derivatives; and

(ii) a second compartment contains a composition containing:

b) one or more manganese salts or one or more zinc salts,
c) hydrogen peroxide or one or more systems that generate hydrogen peroxide,

iv) a third compartment contains a composition containing:

d) one or more (bi)carbonates or one or more systems that generate (bi)carbonate(s), and
e) one or more basifying agents other than the (bi)carbonate(s),

(iii) a fourth compartment contains a composition containing:

f) one or more salts chosen from titanium salts and scandium salts.

In this other embodiment, at least one of the four compositions is aqueous and the ortho-diphenol derivative(s) may be in powder form.

It is also possible to have a four-compartment kit, the first i) containing a composition comprising a) one or more ortho-diphenol derivatives and b) one or more manganese salts or one or more zinc salts, the second ii) containing a composition comprising c) hydrogen peroxide or a system that generates hydrogen peroxide, and the third iii) containing a composition comprising d) the (bi)carbonate and e) the basifying agent other than the (bi)carbonate, and the fourth iv) containing a composition comprising f) one or more salts chosen from titanium salts and scandium salts. In this other kit, at least one of the compositions is preferentially aqueous. More particularly, this composition contains hydrogen peroxide.

According to one particular embodiment of the invention, the kit comprises three compartments: a first compartment comprising a composition containing a) one or more ortho-diphenol derivatives, b) one or more manganese salts or one or more zinc salts, c) hydrogen peroxide or one or more systems that generate hydrogen peroxide, a second compartment containing d) one or more (bi)carbonates and e) one or more basifying agents other than the (bi)carbonate(s), and a third compartment containing f) one or more salts chosen from titanium salts and scandium salts.

Among the three-compartment kits, it is also possible to have kits that contain, in a first compartment, a composition comprising compounds a), b), d) and e) as defined previously, in a second compartment, a composition comprising c) as defined previously, and, in a third compartment, a composition comprising f) as defined previously.

In these two variants of three-compartment kits, the first composition contained in the first compartment comprising either a), b) and c) or a), b), d) and e) is in powder form and, preferably, the second composition is aqueous.

According to one variant, the device according to the invention also comprises an additional composition comprising one or more treating agents.

The compositions of the device according to the invention are packaged in separate compartments, optionally accompanied by suitable application means, which may be identical or different, such as fine brushes, coarse brushes or sponges.

The device mentioned above may also be equipped with a means for dispensing the desired mixture on the hair, for instance the devices described in patent FR 2 586 913.

As indicated previously, a subject of the present invention is also a cosmetic composition for dyeing keratin fibres, comprising ingredients a), b), c), d), e) and f), it being understood that the pH of the composition is alkaline, i.e. greater than 7 and preferably between 8 and 12. It is particularly between 8 and 10.5.

A subject of the invention is also the use of the said cosmetic dye composition for dyeing keratin fibres.

The present invention also relates to the use of one or more salts chosen from titanium salts and scandium salts as defined previously, for improving the chromaticity of the colour of keratin fibres, in particular human keratin fibres such as the hair, dyed with one or more ortho-diphenol derivatives as defined previously.

Indeed, the present invention deals with the use of one or more titanium salts as defined previously, for improving

the chromaticity of the colour of keratin fibres, in particular human keratin fibres such as the hair, dyed with one or more ortho-diphenol derivatives as defined previously.

The present invention also deals with the use of one or more scandium salts as defined previously, for improving the chromaticity of the colour of keratin fibres, in particular human keratin fibres such as the hair, dyed with one or more ortho-diphenol derivatives as defined previously.

In particular, the invention relates to the use of one or more salts chosen from titanium salts and scandium salts in a dye composition comprising one or more ortho-diphenol derivatives, for improving the chromaticity of the colour of keratin fibres.

For the purposes of the present invention, the term "build-up" of the colour of keratin fibres means the variation in coloration between locks of undyed grey hair and locks of dyed hair.

The examples that follow serve to illustrate the invention without, however, being limiting in nature.

Dyeing example I

In the example that follows, the colour build-up ΔE* and the chromaticity C* were compared between a dyeing process according to the invention and a dyeing process (comparative) that is identical but that does not involve the use of a composition comprising titanium salts.

1. Test compositions

| Compositions | A1 | A2 | A3 | A4 |
|---|---|---|---|---|
| Catechin | 0.420 g | - | - | - |
| Haematoxylin | - | 0.420 g | - | - |
| Gallic acid | - | - | 0.420 g | - |
| Quercetin | - | - | - | 0.420 g |
| Manganese gluconate | 0.003 g | 0.003 g | 0.003 g | 0.003 g |
| Hydrogen peroxide | 1.2 g | 1.2 g | 1.2 g | 1.2 g |
| Ethanol | 13.2 g | 13.2 g | 13.2 g | 13.2 g |
| Propylene glycol | 13.2 g | 13.2 g | 13.2 g | 13.2 g |
| Water | qs 100 g | qs 100 g | qs 100 g | qs 100 g |
| (*) 0.144 g of 167-volume aqueous hydrogen peroxide solution | | | | |

| Composition B (revealer) | Amount |
|---|---|
| Sodium bicarbonate | 2.6 g |
| Monoethanolamine | 2 g |
| Demineralized water | qs 100 g |

| Composition C (post-treatment) | Amount |
|---|---|
| Potassium titanium oxalate dihydrate $K_2TiO(C_2O_4)_2$-$2H_2O$ | 0.47 g |
| Demineralized water | qs 100 g |

2. Procedure:

Each composition A1, A2, A3 and A4 is applied to locks of permanent-waved grey hair containing 90% white hairs, at a rate of 12 g of composition per 1 g of hair. The composition is then left to stand on the locks for 30 minutes

at a temperature of 50°C.

After this, the impregnated hair is wiped using an absorbent paper towel to remove the excess composition.

Composition B is then applied to each of the locks at a rate of 4 g of composition per 1 g of lock. The pH of composition B is 9.5. The leave-on time is 10 minutes at room temperature. The hair is then rinsed with water, washed with a standard shampoo and dried under a hood.

Composition C is then applied to each of locks at a rate of 4 g of composition per 1 g of lock; the leave-on time is 10 minutes at room temperature. The hair is then rinsed with water, washed with a standard shampoo and dried under a hood.

3. Colorimetric results:

The colour build-up ΔE* and the chromaticity C* are compared between a dyeing process according to the invention and a process that is identical but that uses water instead of composition C based on titanium salts.

The colour of the locks was evaluated in the CIE L* a* b* system, using a Minolta CM2600D spectrocolorimeter. In this L* a* b* system, the three parameters denote, respectively, the intensity (L*), a* indicates the green/red colour axis and b* the blue/yellow colour axis.

The variation in coloration between the locks of permanent-waved grey hair containing 90% white hairs (90 PWG) that are untreated (control) and after treatment is defined by (ΔE*) according to the following equation:

$$\Delta E^* = \sqrt{(L^* - L_o^*)^2 + (a^* - a_o^*)^2 + (b^* - b_o^*)^2}$$

In this equation, L*, a* and b* represent the values measured on locks of hair after dyeing and $L_0$*, $a_0$* and $b_0$* represent the values measured on locks of untreated undyed hair. The greater the value of ΔE*, the better the colour build-up.

The chromaticity was also measured by the values a* and b* and is obtained from the following formula:

$$C^* = [a^{*2} + b^{*2}]^{1/2}$$

The greater the value of C*, the more chromatic the colour obtained.

The colour build-up ΔE* and chromaticity C* values are given in the tables below:

Table 1: Chromaticity C* results for catechin

| Chromaticity | C* |
|---|---|
| Without titanium salts (comparative) | 19.8 |
| With titanium salts | 21.6 |

Table 2: Chromaticity C* results for haematoxylin

| Chromaticity | C* |
|---|---|
| Without titanium salts (comparative) | 10.9 |
| With titanium salts | 12.1 |

Table 3: Chromaticity C* and colour build-up results for quercetin and gallic acid

| Ingredients a) | Quercetin | | Gallic acid | |
|---|---|---|---|---|
| | ΔE* | C* | ΔE* | C* |
| Without titanium salts (comparative ) | 11.9 | 18.5 | 12.2 | 16.9 |
| With titanium salts (invention) | 14.7 | 21.3 | 13.8 | 17.9 |

It is found that the dyeing process according to the invention gives more chromatic colorations irrespective of the ortho-diphenol derivative used, only when the post-treatment with titanium salts is not performed.
Moreover, the dyeing process according to the invention leads to satisfactory colour build-up for quercetin and gallic acid.

Dyeing example II

In the example that follows, the colour build-up ΔE* and the chromaticity C* were compared between a dyeing process according to the invention and a dyeing process that is identical but that does not involve the use of a composition comprising scandium salts.

1. Test compositions

| Compositions | A'1 | A'2 | A'3 |
|---|---|---|---|
| Catechin a) | 0.420 g | - | - |
| Haematoxylin a) | - | 0.420 g | - |
| Quercetin a) | - | - | 0.420 g |
| Manganese gluconate b) | 0.003 g | 0.003 g | 0.003 g |
| Hydrogen peroxide* c) | 1.2 g | 1.2 g | 1.2 g |
| Ethanol | 13.2 g | 13.2 g | 13.2 g |
| Propylene glycol | 13.2 g | 13.2 g | 13.2 g |
| Water | qs 100 g | qs 100 g | qs 100 g |
| (*) 0.144 g of 167-volume aqueous hydrogen peroxide solution | | | |

| Composition B (revealer) | Amount |
|---|---|
| Sodium bicarbonate d) | 2.6 g |
| Monoethanolamine e) | 2 g |
| Demineralized water | qs 100 g |

| Composition C (post-treatment) | Amount |
|---|---|
| $Sc(OAc)_3$ f) | 0.40 g |
| Demineralized water | qs 100 g |

2. Procedure:

Each composition A'1, A'2, and A'3 is applied to locks of permanent-waved grey hair containing 90% white hairs, at a rate of 12 g of composition per 1 g of hair. The composition is then left to stand on the locks for 30 minutes at a temperature of 50°C.
After this, the impregnated hair is wiped using an absorbent paper towel to remove the excess composition.
Composition B is then applied to each of the locks at a rate of 4 g of composition per 1 g of lock. The pH of composition B is 9.5. The leave-on time is 10 minutes at room temperature. The hair is then rinsed with water, washed with a standard shampoo and dried under a hood.
Composition C is then applied to each of locks at a rate of 4 g of composition per 1 g of lock; the leave-on time is 10 minutes at room temperature. The hair is then rinsed with water, washed with a standard shampoo and dried under a hood.

3. Colorimetric results:

The colour build-up ΔE* and the chromaticity C* are compared between a dyeing process according to the invention and a process that is identical but that uses water instead of composition C based on scandium salts.

The colour of the locks was evaluated in the CIE L* a* b* system, using a Minolta CM2600D spectrocolorimeter. In this L* a* b* system, the three parameters denote, respectively, the intensity (L*), a* indicates the green/red colour axis and b* the blue/yellow colour axis.

The variation in coloration between the locks of permanent-waved grey hair containing 90% white hairs (90 PWG) that are untreated (control) and after treatment is defined by (ΔE*) according to the following equation:

$$\Delta E^* = \sqrt{(L^* - L_o^*)^2 + (a^* - a_o^*)^2 + (b^* - b_o^*)^2}$$

In this equation, L*, a* and b* represent the values measured on locks of hair after dyeing and $L_0$*, $a_0$* and $b_0$* represent the values measured on locks of untreated undyed hair. The greater the value of ΔE*, the better the colour build-up.

The chromaticity was also measured by the values a* and b* and is obtained from the following formula:

$$C^* = \sqrt{a^{*2} + b^{*2}}$$

The greater the value of C*, the more chromatic the colour obtained.

The colour build-up ΔE* and chromaticity C* values are given in the tables below:

| Ingredients a) | Catechin | | Quercetin | | Haematoxylin | |
|---|---|---|---|---|---|---|
| | ΔE* | C* | ΔE* | C* | ΔE* | C* |
| Without scandium salts (comparative) | 22 | 19.8 | 11.9 | 18.5 | 26.5 | 10.9 |
| With scandium salts (invention) | 22.8 | 24.3 | 13.9 | 20.7 | 27.8 | 13.0 |

It is found that the dyeing process according to the invention gives more chromatic colorations irrespective of the ortho-diphenol derivative used.

Moreover, the dyeing process according to the invention leads to satisfactory colour build-up.

It is also seen visually that the process of the invention gives better colour build-up and better chromaticity of the coloration than those obtained with the comparative process. This is confirmed by the colorimetric measurements, which demonstrate that the dyeing process according to the invention gives more chromatic colorations C* and more satisfactory colour build-up irrespective of the ortho-diphenol derivative used, when compared with a process not using a post-treatment with a scandium salt.

## Claims

1. Process for dyeing keratin fibres, in particular human keratin fibres such as the hair, in which the said fibres are treated with one or more cosmetic compositions containing, taken together or separately in the said composition(s), the following ingredients:

   a) one or more ortho-diphenol derivatives,
   b) one or more manganese salts or one or more zinc salts,
   c) hydrogen peroxide or one or more systems that generate hydrogen peroxide,
   d) one or more (bi)carbonates or one or more systems that generate (bi)carbonate(s), and
   e) one or more basifying agents other than the (bi)carbonate(s), and
   f) one or more salts chosen from titanium salts and scandium salts;

   it being understood that the pH of at least one of the compositions comprising at least one of the ingredients a), b), d), e) and/or f) is alkaline, i.e. greater than 7, and that the composition comprising f) the salt(s) chosen from titanium

salts and scandium salts is applied to the keratin fibres as the last step of the dyeing process.

2. Dyeing process according to Claim 1, **characterized in that** the ortho-diphenol derivative(s) are chosen from natural ortho-diphenol derivatives.

3. Dyeing process according to Claim 1, **characterized in that** ingredient a) is an ortho-diphenol bearing an aromatic ring chosen from benzene, naphthalene, tetrahydronaphthalene, indane, indene, anthracene, phenanthrene, isoindole, indoline, isoindoline, benzofuran, dihydrobenzofuran, chroman, isochroman, chromene, isochromene, quinoline, tetrahydroquinoline and isoquinoline, the said aromatic ring comprising at least two hydroxyl groups borne by two adjacent contiguous carbon atoms of the aromatic ring.

4. Dyeing process according to any one of the preceding claims, **characterized in that** ingredient a) is an ortho-diphenol of formula (I) below or an oligomer thereof, in salified or non-salified form:

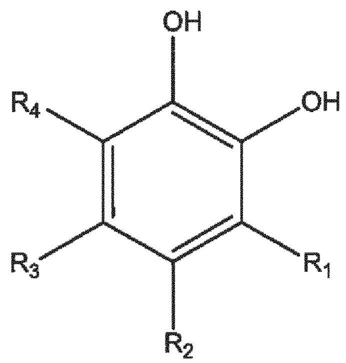

(I)

in which formula (I) the substituents:

• $R_1$ to $R_4$, which may be identical or different, represent:

- a hydrogen atom;
- a halogen atom,
- a hydroxyl radical,
- a carboxyl radical,
- an alkyl carboxylate or alkoxycarbonyl radical,
- an optionally substituted amino radical,
- an optionally substituted linear or branched alkyl radical,
- an optionally substituted linear or branched alkenyl radical,
- an optionally substituted cycloalkyl radical,
- an alkoxy radical,
- an alkoxyalkyl radical,
- an alkoxyaryl radical, the aryl group possibly being optionally substituted,
- an aryl radical,
- a substituted aryl radical,
- a saturated or unsaturated heterocyclic radical, optionally bearing a cationic or anionic charge, optionally substituted and/or optionally fused with an aromatic ring, the said aromatic ring being optionally substituted,
- a radical containing one or more silicon atoms,

• in which two of the substituents borne by two adjacent carbon atoms $R_1$ - $R_2$, $R_2$ - $R_3$ or $R_3$ - $R_4$ form, together with the carbon atoms that bear them, a saturated or unsaturated, aromatic or non-aromatic ring, optionally containing one or more heteroatoms and optionally fused with one or more saturated or unsaturated rings optionally containing one or more heteroatoms.

5. Dyeing process according to any one of the preceding claims, **characterized in that** the ortho-diphenol derivative(s) are chosen from:

- flavonols,

- anthocyanidins,
- anthocyanins or anthocyans,
- ortho-hydroxybenzoates,
- flavones,
- hydroxystilbenes,
- 3,4-dihydroxyphenylalanine and derivatives thereof,
- 2,3-dihydroxyphenylalanine and derivatives thereof,
- 4,5-dihydroxyphenylalanine and derivatives thereof,
- dihydroxycinnamates,
- ortho-polyhydroxycoumarins,
- ortho-polyhydroxyisocoumarins,
- ortho-polyhydroxycoumarones,
- ortho-polyhydroxyisocoumarones,
- ortho-polyhydroxychalcones,
- ortho-polyhydroxychromones,
- ortho-polyhydroxyquinones,
- ortho-hydroxyxanthones,
- 1,2 dihydroxybenzene and derivatives thereof,
- 1,2,4-trihydroxybenzene and derivatives thereof,
- 1,2,3-trihydroxybenzene and derivatives thereof,
- 2,4,5-trihydroxytoluene and derivatives thereof,
- proanthocyanidins,
- proathocyanins,
- tannic acid,
- ellagic acid,
- and mixtures of the preceding compounds,

**6.** Dyeing process according to any one of Claims 2 to 5, **characterized in that** the natural ortho-diphenol derivative(s) are chosen from animal, bacterial, fungal, algal, plant and fruit extracts.

**7.** Dyeing process according to any one of the preceding claims, **characterized in that**:

- the manganese salts are chosen from:

  i) manganese oxides and
  ii) halides, sulfates, phosphates, nitrates and perchlorates, carboxylic acid salts such as gluconates, and also mixtures thereof, and

- the zinc salts are chosen from zinc sulfate, zinc gluconate, zinc chloride, zinc lactate, zinc acetate, zinc glycinate and zinc aspartate;

and more particularly, the salts b) are chosen from carboxylic acid salts of manganese.

**8.** Dyeing process according to any one of the preceding claims, **characterized in that** ingredient c) is hydrogen peroxide or urea peroxide.

**9.** Dyeing process according to any one of the preceding claims, **characterized in that** the (bi)carbonate(s) are alkali metal, alkaline-earth metal or ammonium (bi)carbonates.

**10.** Dyeing process according to any one of the preceding claims, **characterized in that** the alkaline agent(s) other than the (bi)carbonate(s) are chosen from:

- aqueous ammonia,
- alkanolamines,
- oxyethylenated ethylenediamines,
- oxypropylenated ethylenediamines,
- mineral or organic hydroxides,
- alkali metal silicates,

- amino acids, and
- the compounds of formula (II) below:

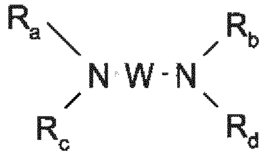

(II)

in which formula (II):
- W is a divalent ($C_1$-$C_8$)alkylene group, preferably propylene optionally substituted especially with a hydroxyl group or a $C_1$-$C_4$ alkyl radical;
- $R_a$, $R_b$, $R_c$ and $R_d$, which may be identical or different, represent a hydrogen atom or a $C_1$-$C_4$ alkyl or $C_1$-$C_4$ hydroxyalkyl radical. preferably, the alkaline agent(s) e) are chosen from alkanolamines, in particular monoethanolamine.

**11.** Dyeing process according to any one of the preceding claims, **characterized in that** the titanium derivative(s) are chosen from:

i) titanium IV salts such as potassium titanium oxalate dihydrate $K_2TiO(C_2O_4)_2$-$2H_2O$, $Ti(SO_4)_2$,
ii)titanium oxides and salts thereof, hydrates thereof and supported forms thereof.

**12.** Dyeing process according to any one of Claims 1 to 10, **characterized in that** scandium salts are chosen from scandium hydroxide, scandium halides such as scandium chloride, scandium sulfate, scandium nitrate, carboxylic acid salts of scandium such as anhydrous or hydrated scandium acetate, and (poly)halo($C_1$-$C_6$)alkylsulfonates such as scandium trifluoromethanesulfonate.

**13.** Process according to any one of the preceding claims, **characterized in that** it is performed in one step, which consists in applying to keratin fibres an aqueous composition comprising a), b), c), d), e) and f) as defined according to any one of the preceding claims.

**14.** Process according to any one of Claims 1 to 12, **characterized in that** the first step consists in applying to the said fibres a composition comprising ingredients a), b) and c) as defined according to any one of Claims 1 to 8, and then, in a second step, a composition comprising ingredients d) and e) as defined according to Claims 9 and 10, and then, in a third step, a composition comprising ingredient f) as defined according to Claim 11 or 12 is applied to the said fibres, it being understood that at least one of the three compositions is aqueous.

**15.** Process according to any one of the preceding claims, in which mechanical wiping and/or drying and/or rinsing of the keratin fibres is performed before performing the step comprising the application of a composition comprising ingredient d) and/or e) as defined according to any one of Claims 1, 9 and 10.

**16.** Cosmetic composition for dyeing keratin fibres, containing compounds a), b), c), d), e) and f) as defined according to any one of Claims 1 to 12.

**17.** Multi-compartment device comprising from 2 to 6 compartments containing from 2 to 6 compositions, in which are distributed ingredients a), b), c), d), e) and f) as defined in any one of Claims 1 to 12, the said compositions being aqueous or pulverulent, with at least one of these compositions being aqueous.

**18.** Use of one or more salts chosen from titanium salts and scandium salts as defined in Claims 1, 11 and 12, for improving the chromaticity and/or the colour build-up of keratin fibres, in particular human keratin fibres such as the hair, dyed with one or more ortho-diphenol derivatives as defined in any one of Claims 1 to 6.

## Patentansprüche

**1.** Verfahren zum Färben von Keratinfasern, insbesondere menschlichen Keratinfasern wie dem Haar, bei dem man die Fasern mit einer oder mehreren kosmetischen Zusammensetzungen behandelt, die zusammengenommen oder

separat in der Zusammensetzung bzw. den Zusammensetzungen die folgenden Bestandteile enthalten:

a) ein oder mehrere ortho-Diphenol-Derivate,
b) ein oder mehrere Mangansalze oder ein oder mehrere Zinksalze,
c) Wasserstoffperoxid oder ein oder mehrere Wasserstoffperoxid erzeugende Systeme,
d) ein oder mehrere (Hydrogen)carbonate oder ein oder mehrere (Hydrogen)carbonat(e) erzeugende Systeme und
e) ein oder mehrere Alkalinisierungsmittel, die von dem bzw. den (Hydrogen) carbanat (en) verschieden sind, und
f) ein oder mehrere Salze, die aus Titansalzen und Scandiumsalzen ausgewählt sind;

wobei es sich versteht, dass der pH-Wert mindestens einer der Zusammensetzungen, die mindestens einen der Bestandteile a), b), d), e) und/oder f) umfassen, alkalisch, d.h. größer als 7, ist und dass die Zusammensetzung, die f) das Salz bzw. die Salze, das bzw. die aus Titansalzen und Scandiumsalzen ausgewählt ist bzw. sind, als letzter Schritt des Färbeverfahrens auf die Keratinfasern aufgebracht wird.

2. Färbeverfahren nach Anspruch 1, **dadurch gekennzeichnet, dass** das ortho-Diphenol-Derivat bzw. die ortho-Diphenol-Derivate aus natürlichen ortho-Diphenol-Derivaten ausgewählt ist bzw. sind.

3. Färbeverfahren nach Anspruch 1, **dadurch gekennzeichnet, dass** es sich bei Bestandteil a) um ein ortho-Diphenol mit einem aromatischen Ring handelt, das aus Benzol, Naphthalin, Tetrahydronaphthalin, Indan, Inden, Anthracen, Phenanthren, Isoindol, Indolin, Isoindolin, Benzofuran, Dihydrobenzofuran, Chroman, Isochroman, Chromen, Isochromen, Chinolin, Tetrahydrochinolin und Isochinolin ausgewählt ist, wobei der aromatische Ring mindestens zwei Hydroxylgruppen an zwei benachbarten zusammenhängenden Kohlenstoffatomen des aromatischen Rings umfasst.

4. Färbeverfahren nach einem der vorhergehenden Ansprüche, **dadurch gekennzeichnet, dass** es sich bei Bestandteil a) um ein ortho-Diphenol der nachstehenden Formel (I) oder ein Oligomer davon in versalzter oder nicht versalzter Form handelt:

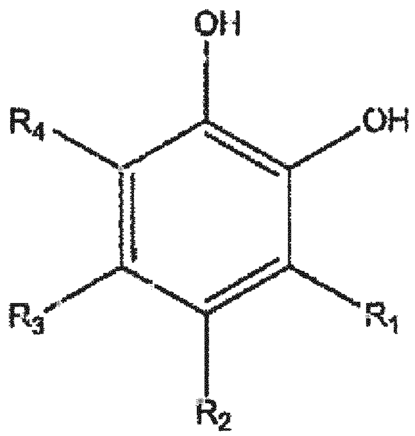

(I)

wobei in der Formel (I) die Substituenten:

• $R_1$ bis $R_4$, die gleich oder verschieden sein können, für:

- ein Wasserstoffatom,
- ein Halogenatom,
- einen Hydroxylrest,
- einen Carboxylrest,
- einen Alkylcarboxylat- oder Alkoxycarbonylrest,
- einen gegebenenfalls substituierten Aminorest,
- einen gegebenenfalls substituierten linearen oder verzweigten Alkylrest,
- einen gegebenenfalls substituierten linearen oder verzweigten Alkenylrest,
- einen gegebenenfalls substituierten Cycloalkylrest,
- einen Alkoxyrest,

- einen Alkoxyalkylrest,
- einen Alkoxyarylrest, wobei die Arylgruppe gegebenenfalls substituiert ist
- einen Arylrest,
- einen substituierten Arylrest,
- einen gesättigten oder ungesättigten heterocyclischen Rest, der gegebenenfalls eine kationische oder anionische Ladung trägt, gegebenenfalls substituiert und/oder gegebenenfalls mit einem aromatischen Ring anelliert ist, wobei der aromatische Ring gegebenenfalls substituiert ist,
- einen Rest, der ein oder mehrere Siliciumatome enthält,

stehen,
• wobei zwei der Substituenten der zwei benachbarten Kohlenstoffatome $R_1$ - $R_2$, $R_2$ - $R_3$ or $R_3$ - $R_4$ zusammen mit den Kohlenstoffatomen, die sie tragen, einen gesättigten oder ungesättigten, aromatischen oder nichtaromatischen Ring bilden, der gegebenenfalls ein oder mehrere Heteroatome enthält und gegebenenfalls mit einem oder mehreren gesättigten oder ungesättigten Ringen, die gegebenenfalls ein oder mehrere Heteroatome enthalten, anelliert ist.

**5.** Färbeverfahren nach einem der vorhergehenden Ansprüche, **dadurch gekennzeichnet, dass** das ortho-Diphenol-Derivat bzw. die ortho-Diphenol-Derivate aus:

- Flavonolen,
- Anthocyanidinen,
- Anthocyaninen oder Anthocyanen,
- ortho-Hydroxybenzoaten,
- Flavonen,
- Hydroxystilbenen,
- 3,4-Dihydroxyphenylalanin und Derivaten davon,
- 2,3-Dihydroxyphenylalanin und Derivaten davon,
- 4,5-Dihydroxyphenylalanin und Derivaten davon,
- Dihydroxycinnamaten,
- ortho-Polyhydroxycumarinen,
- ortho-Polyhydroxyisocumarinen,
- ortho-Polyhydroxycumaronen,
- ortho-Polyhydroxyisocumaronen,
- ortho-Polyhydroxychalconen,
- ortho-Polyhydroxychromonen,
- ortho-Polyhydroxychinonen,
- ortho-Hydroxyxanthonen,
- 1,2-Dihydroxybenzol und Derivaten davon,
- 1,2,4-Trihydroxybenzol und Derivaten davon,
- 1,2,3-Trihydroxybenzol und Derivaten davon,
- 2,4,5-Trihydroxytoluol und Derivaten davon,
- Proanthocyanidinen,
- Proathocyaninen,
- Tanninsäure,
- Ellagsäure
- und Mischungen der vorstehenden Verbindungen ausgewählt ist bzw. sind.

**6.** Färbeverfahren nach einem der Ansprüche 2 bis 5, **dadurch gekennzeichnet, dass** das natürliche ortho-Diphenol-Derivat bzw. die natürlichen ortho-Diphenol-Derivate aus Tier-, Bakterien-, Pilz-, Algen-, Pflanzen- und Fruchtextrakten ausgewählt ist bzw. sind.

**7.** Färbeverfahren nach einem der vorhergehenden Ansprüche, **dadurch gekennzeichnet, dass**:

- die Mangansalze aus:

i) Manganoxiden und
ii) Halogeniden, Sulfaten, Phosphaten, Nitraten und Perchloraten, Carbonsäuresalzen wie Gluconaten sowie Mischungen davon ausgewählt sind und

- die Zinksalze aus Zinksulfat, Zinkgluconat, Zinkchlorid, Zinklactat, Zinkacetat, Zinkglycinat und Zinkaspartat ausgewählt sind;

und spezieller die Salze b) aus Carbonsäuresalzen des Mangans ausgewählt sind.

**8.** Färbeverfahren nach einem der vorhergehenden Ansprüche, **dadurch gekennzeichnet, dass** es sich bei Bestandteil c) um Wasserstoffperoxid oder Harnstoffperoxid handelt.

**9.** Färbeverfahren nach einem der vorhergehenden Ansprüche, **dadurch gekennzeichnet, dass** es sich bei dem bzw. den (Hydrogen) carbonat (en) um Alkalimetall-, Erdalkalimetall- oder Ammonium(hydrogen)-carbonate handelt.

**10.** Färbeverfahren nach einem der vorhergehenden Ansprüche, **dadurch gekennzeichnet, dass** das alkalische Mittel bzw. die alkalischen Mittel, das bzw. die von dem bzw. den (Hydrogen) carbonat (en) verschieden ist bzw. sind, aus:

- wässrigem Ammoniak,
- Alkanolaminen,
- oxyethylenierten Ethylendiaminen,
- oxypropylenierten Ethylendiaminen,
- mineralischen oder organischen Hydroxiden,
- Alkalimetallsilikaten
- Aminosäuren und
- den Verbindungen der nachstehenden Formel (II):

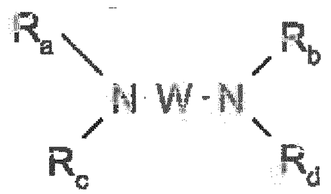

(II)

wobei in Formel (II):
- W für eine zweiwertige ($C_1$-$C_8$) -Alkylengruppe, vorzugsweise Propylen, insbesondere gegebenenfalls substituiert durch eine Hydroxylgruppe oder einen $C_1$-$C_4$-Alkylrest, steht;
- $R_a$, $R_b$, $R_c$ und $R_d$, die gleich oder verschieden sein können, für ein Wasserstoffatom oder einen $C_1$-$C_4$-Alkyl- oder $C_1$-$C_4$-Hydroxyalkylrest stehen;

ausgewählt ist bzw, sind, wobei das alkalische Mittel bzw. die alkalischen Mittel e) vorzugsweise aus Alkanolaminen, insbesondere Monoethanolamin, ausgewählt ist bzw. sind.

**11.** Färbeverfahren nach einem der vorhergehenden Ansprüche, **dadurch gekennzeichnet, dass** das Titanderivat bzw. die Titanderivate aus:

i) Titan-IV-Salzen wie Kaliumtitanoxalatdihydrat $K_2TiO(C_2O_4)_2$-$2H_2O$, $Ti(SO_4)_2$,
ii) Titanoxiden und Salzen davon, Hydraten davon und geträgerten Formen davon

ausgewählt ist bzw. sind.

**12.** Färbeverfahren nach einem der Ansprüche 1 bis 10, **dadurch gekennzeichnet, dass** die Scandiumsalze aus Scandiumhydroxid, Scandiumhalogeniden wie Scandiumchlorid, Scandiumsulfat, Scandiumnitrat, Carbonsäuresalzen von Scandium wie wasserfreiem oder hydratisiertem Scandiumacetat und (Poly)-halogen($C_1$-$C_6$)alkylsulfonaten wie Scandiumtrifluormethansulfonat ausgewählt sind.

**13.** Verfahren nach einem der vorhergehenden Ansprüche, **dadurch gekennzeichnet, dass** es in einem Schritt durchgeführt wird, der darin besteht, dass man auf Keratinfasern eine wässrige Zusammensetzung, die a), b), c), d), e) und f) gemäß der in einem der vorhergehenden Ansprüche angegebenen Definition aufbringt.

**14.** Verfahren nach einem der Ansprüche 1 bis 12, **dadurch gekennzeichnet, dass** der erste Schritt darin besteht,

dass man auf die Fasern eine Zusammensetzung, die die Bestandteile a), b) und c) gemäß der in einem der Ansprüche 1 bis 8 angegebenen Definition umfasst, aufbringt und dann in einem zweiten Schritt eine Zusammensetzung, die die Bestandteile d) und e) gemäß der in den Ansprüchen 9 und 10 angegebenen Definition umfasst, und dann in einem dritten Schritt eine Zusammensetzung, die Bestandteil f) gemäß der in Anspruch 11 oder 12 angegebenen Definition umfasst, auf die Fasern aufbringt, wobei sich versteht, dass mindestens eine der drei Zusammensetzungen wässrig ist.

**15.** Verfahren nach einem der vorhergehenden Ansprüche, bei dem vor der Durchführung des Schritts, der das Aufbringen einer Zusammensetzung, die den Bestandteil d) und/oder e) gemäß der in einem der Ansprüche 1, 9 und 10 angegebenen Definition umfasst, umfasst die Keratinfasern mechanisch abgewischt und/oder getrocknet und/oder gespült werden.

**16.** Kosmetische Zusammensetzung zum Färben von Keratinfasern, die die Verbindungen a), b), c), d), e) und f) gemäß der in einem der Ansprüche 1 bis 12 angegebenen Definition enthält.

**17.** Vorrichtung mit mehreren Kompartimenten, umfassend 2 bis 6 Kompartimente, die 2 bis 6 Zusammensetzungen enthalten, in denen die Bestandteile a), b), c), d) e) und f) gemäß der in einem der Ansprüche 1 bis 12 angegebenen Definition verteilt sind, wobei die Zusammensetzungen wässrig oder pulverförmig sind, wobei mindestens eine dieser Zusammensetzungen wässrig ist.

**18.** Verwendung eines oder mehrerer Salze, die aus Titansalzen und Scandiumsalzen gemäß der in einem der Ansprüche 1, 11 und 12 angegebenen Definition ausgewählt sind, zur Verbesserung der Farbart und/oder des Farbaufbaus von mit einem oder mehreren ortho-Diphenol-Derivaten gemäß der in einem der Ansprüche 1 bis 6 angegebenen Definition gefärbten Keratinfasern, insbesondere menschlichen Keratinfasern, wie dem Haar.

## Revendications

**1.** Procédé de coloration des fibres kératiniques, en particulier des fibres kératiniques humaines telles que les cheveux, dans lequel lesdites fibres sont traitées par une ou plusieurs compositions cosmétiques contenant, pris ensemble ou séparément dans la ou lesdites compositions, les ingrédients suivants :

a) un ou plusieurs dérivé(s) d'orthodiphénol(s),
b) un ou plusieurs sel(s) de manganèse ou un ou plusieurs sel(s) de zinc,
c) du peroxyde d'hydrogène ou un ou plusieurs système(s) générateur(s) de peroxyde d'hydrogène,
d) un ou plusieurs (bi)carbonate(s) ou un ou plusieurs système(s) générateur(s) de (bi)carbonate(s), et
e) un ou plusieurs agent(s) alcalin(s) différent(s) du ou de(s) bicarbonate(s), et
f) un ou plusieurs sel(s) choisi(s) parmi les sels de titane et les sels de scandium;

étant entendu que le pH d'au moins une des compositions comprenant l'un au moins des ingrédients a), b), d), e) et/ou f) est alcalin, soit supérieur à 7, et que la composition comprenant f) le ou les sels choisis parmi les sels de titane et les sels de scandium est appliquée sur les fibres kératiniques en dernière étape du procédé de coloration.

**2.** Procédé de coloration selon la revendication 1, **caractérisé en ce que** le ou les dérivé(s) d'orthodiphénol(s) sont choisis parmi les dérivé(s) d'orthodiphénol(s) naturel(s).

**3.** Procédé de coloration selon la revendication 1 ou 2, **caractérisé en ce que** l'ingrédient a) est un orthodiphénol à cycle aromatique choisi parmi le benzène, le naphtalène, le tétrahydronaphtalène, l'indane, l'indène, l'anthracène, le phénanthrène, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine, ledit cycle aromatique comportant au moins deux groupes hydroxy portés par deux atomes adjacents contigus du cycle aromatique.

**4.** Procédé de coloration selon l'une quelconque des revendications précédentes, **caractérisé en ce que** l'ingrédient a) est un orthodiphénol de formule (I) suivante ou l'un de ses oligomères, sous forme salifiée ou non :

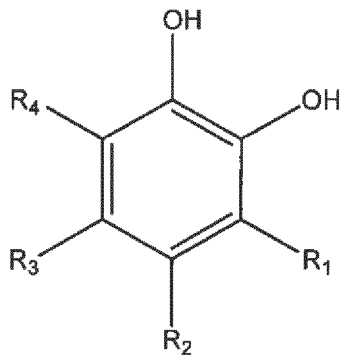

(I)

formule (I) dans laquelle les substituants :

- $R_1$ à $R_4$, identiques ou différents, représentent :
  - un atome d'hydrogène,
  - un atome halogène,
  - un radical hydroxy,
  - un radical carboxyle,
  - un radical carboxylate d'alkyle ou alcoxycarbonyle,
  - un radical amino éventuellement substitué,
  - un radical alkyle linéaire ou ramifié éventuellement substitué,
  - un radical alcényle linéaire ou ramifié éventuellement substitué,
  - un radical cycloalkyle éventuellement substitué,
  - un radical alcoxy,
  - un radical alcoxyalkyle,
  - un radical alcoxyaryle, le groupe aryle pouvant être éventuellement substitué,
  - un radical aryle,
  - un radical aryle substitué,
  - un radical hétérocyclique, saturé ou non, porteur ou non d'une charge cationique ou anionique, éventuellement substitué et/ou éventuellement condensé avec un cycle aromatique, ledit cycle aromatique étant éventuellement substitué,
  - un radical contenant un ou plusieurs atomes de silicium.

    - ou deux des substituants portés par deux atomes de carbone adjacents $R_1$ - $R_2$, $R_2$ - $R_3$ ou $R_3$ - $R_4$ forment conjointement avec les atomes de carbone les portant un cycle saturé ou insaturé, aromatique ou non, contenant éventuellement un ou plusieurs hétéroatomes et éventuellement condensé avec un ou plusieurs cycles saturés ou insaturés contenant éventuellement un ou plusieurs hétéroatomes.

**5.** Procédé de coloration selon l'une quelconque des revendications précédentes, **caractérisé en ce que** le ou les dérivé(s) orthodiphénol(s) sont choisis parmi :

- les flavonols,
- les anthocyanidines,
- les anthocyanines ou les anthocyanes,
- les orthohydroxybenzoates,
- les flavones,
- les hydroxystilbènes,
- la 3,4-dihydroxyphénylalanine et ses dérivées,
- la 2,3-dihydroxyphénylalanine et ses dérivés,
- la 4,5-dihydroxyphénylalanine et ses dérivés,
- les dihydroxycinnamates,
- les orthopolyhydroxycoumarines,
- les orthopolyhydroxyisocoumarines,
- les orthopolyhydroxycoumarones,
- les orthopolyhydroxyisocoumarones,

- les orthopolyhydroxychalcones,
- les orthopolyhydroxychromones,
- les orthopolyhydroxyquinones,
- les orthohydroxyxanthones,
- le 1,2 dihydroxybenzène et ses dérivés,
- le 1,2,4 trihydroxybenzène et ses dérivés,
- le 1,2,3 trihydroxybenzène et ses dérivés,
- le 2,4,5 trihydroxytoluène et ses dérivés,
- les proanthocyanidines,
- les proathocyanines,
- l'acide tannique,
- l'acide ellagique,
- et les mélanges des composés précédents.

**6.** Procédé de coloration selon l'une quelconque des revendications 2 à 5, **caractérisé en ce que** le ou les dérivé(s) orthodiphénol(s) naturel(s) sont choisis parmi les extraits d'animaux, de bactéries, de champignons, d'algues, de plantes et de fruits.

**7.** Procédé de coloration selon l'une quelconque des revendications précédentes, **caractérisé en ce que** :

- les sels de manganèse sont choisis parmi :

  i) les oxydes de manganèse et
  ii) les halogénures, les sulfates, phosphates, nitrates et perchlorates, les sels d'acides carboxyliques tels que les gluconates ainsi que leurs mélanges, et

- les sels de zinc sont choisis parmi le sulfate de zinc, le gluconate de zinc, le chlorure de zinc, le lactate de zinc, l'acétate de zinc, le glycinate de zinc et l'aspartate de zinc ;

et plus particulièrement, les sels b) sont choisis parmi les sels d'acide carboxyliques de manganèse.

**8.** Procédé de coloration selon l'une quelconque des revendications précédentes, **caractérisé en ce que** l'ingrédient c) est du peroxyde d'hydrogène ou du peroxyde d'urée.

**9.** Procédé de coloration selon l'une quelconque des revendications précédentes, **caractérisé en ce que** le ou les (bi)carbonates sont des (bi)carbonates alcalins, alcalino-terreux ou d'ammonium.

**10.** Procédé de coloration selon l'une quelconque des revendications précédentes, **caractérisé en ce que** le ou les agents alcalins différents du ou de(s) bicarbonate(s) sont choisis parmi :

- l'ammoniaque,
- les alcanolamines,
- les éthylènediamines oxyéthylénées
- les éthylènediamines oxypropylénées,
- les hydroxydes minéraux ou organiques,
- les silicates de métaux alcalins,
- les acides aminés, et
- les composés de formule (II) suivante :

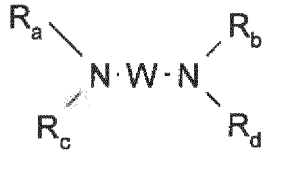

(II)

formule (II) dans laquelle :
- **W** est un groupe divalent $(C_1-C_8)$alkylène, de préférence propylène, éventuellement substitué notamment par

un groupement hydroxy ou un radical alkyle en $C_1$-$C_4$;
- **$R_a$, $R_b$, $R_c$** et **$R_d$**, identiques ou différents, représentent un atome d'hydrogène, un radical alkyle en $C_1$-$C_4$ ou hydroxyalkyle en $C_1$-$C_4$ ;

de préférence, le ou les agents alcalins e) sont choisis parmi les alcanolamines, en particulier la monoéthanolamine.

**11.** Procédé de coloration selon l'une quelconque des revendications précédentes, **caractérisé en ce que** le ou les dérivé(s) du titane sont choisis parmi :

i) les sels de titane IV tels que l'oxalate de titane et potassium dihydrate $K_2TiO(C_2O_4)_2$-$2H_2O$, le $Ti(SO_4)_2$,
ii) les oxydes de titane et leurs sels, leurs hydrates et leurs formes supportées.

**12.** Procédé de coloration selon l'une quelconque des revendications 1 à 10, **caractérisé en ce que** les sels de scandium sont choisis parmi l'hydroxyde de scandium, les halogénures de scandium tels que le chlorure de scandium, le sulfate de scandium, le nitrate de scandium, les sels d'acide carboxylique de scandium tels que l'acétate de scandium hydraté ou non hydraté, et les (poly)halogéno($C_1$-$C_6$)alkylsulfonates tels que le trifluorométhane-sulfonate de scandium.

**13.** Procédé selon l'une quelconque des revendications précédentes, **caractérisé en ce qu'**il est réalisé en une étape consistant à appliquer sur les fibres kératiniques une composition aqueuse comprenant a), b), c), d), e) et f) tels que définis selon l'une quelconque des revendications précédentes.

**14.** Procédé selon l'une quelconque des revendications 1 à 12, **caractérisé en ce que** la première étape consiste à appliquer sur lesdites fibres une composition comprenant les ingrédients a), b) et c) tels que définis selon l'une quelconque des revendications 1 à 8, puis, dans une deuxième étape, une composition comprenant les ingrédients d) et e) tels que définis selon les revendications 9 et 10, puis, dans une troisième étape, une composition comprenant l'ingrédient f) tel que défini selon la revendication 11 ou 12 est appliquée sur lesdites fibres, étant entendu qu'au moins une des trois compositions est aqueuse.

**15.** Procédé selon l'une quelconque des revendications précédentes, dans lequel un essuyage mécanique et/ou un séchage et/ou un rinçage des fibres kératiniques est effectué avant de réaliser l'étape comprenant l'application d'une composition comprenant l'ingrédient d) et/ou e) tels que définis selon l'une quelconque des revendications 1, 9 ou 10.

**16.** Composition cosmétique pour la coloration des fibres kératiniques contenant les composés a), b), c), d), e) et f) tels que définis selon l'une quelconque des revendications 1 à 12.

**17.** Dispositif à plusieurs compartiments comprenant de 2 à 6 compartiments contenant de 2 à 6 compositions, dans lesquels sont répartis les ingrédients a), b), c), d), e) et f) tels que définis selon l'une quelconque des revendications 1 à 12, lesdites compositions étant aqueuses ou pulvérulentes, avec une au moins de ces compositions étant aqueuse.

**18.** Utilisation d'un ou plusieurs sels choisi(s) parmi les sels de titane et les sels de scandium tels que définis dans les revendications 1, 11 et 12, pour améliorer la chromaticité et/ou la montée de la couleur des fibres kératiniques, en particulier des fibres kératiniques humaines telles que les cheveux, colorées à partir d'un ou plusieurs dérivé(s) d'orthodiphénol(s) tels que définis dans une quelconque des revendications 1 à 6.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2814943
- FR 2814945
- FR 2814946
- FR 2814947
- EP 2196182 A
- WO 2011000892 A
- FR 2951374
- US 5008093 A
- US 3376110 A
- US 5183901 A
- US 3931249 A
- FR 2586913


### Non-patent literature cited in the description


- Metal and Dyes. Ullmann's Encyclopaedia. 2005, 8
- Titanium, Titanium Alloys and Titanium Compounds. Ullhamnn encyclopedia. Wiley-VCH Verlag GmbH & Co. KGaA, 2005, 1-33